# EUROPEAN PATENT APPLICATION

(11) **EP 4 495 114 A1**
(43) Date of publication of application: **22.01.2025**
(21) Application number: 23770910.0
(22) Date of filing: 17.03.2023
(51) Int. Cl.: C07D 409/14, A61K 31/4439, A61P 31/10, C07D 413/14

(54) **AZOLE COMPOUND AND ANTIFUNGAL AGENT**

(30) Priority: 18.03.2022 JP 2022043359; 29.11.2022 JP 2022190422
(71) Applicant: Nihon Nohyaku Co., Ltd., Tokyo 104-8386 (JP)
(72) Inventor: MIYAZAKI, Yosuke, Kawachinagano-shi, Osaka 586-0094 (JP); NAKAMURA, Akihiro, Kawachinagano-shi, Osaka 586-0094 (JP)
(74) Representative: Weickmann & Weickmann PartmbB
(86) International application number: PCT/JP2023/010575
(87) International publication number: WO 2023/176957

(57) **Abstract**

The present invention provides compounds which exhibit superior antifungal activity and are used for the treatment of fungal infections such as localized fungal infections and systemic fungal infections, caused by Trichophyton, Candida, Aspergillus and the like.

The present invention relates to an azole compound represented by the general formula (I) 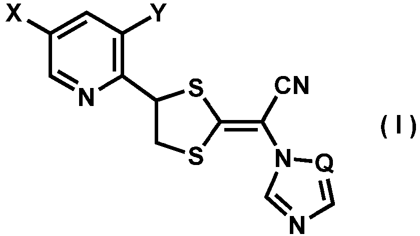 wherein X is a -N(R¹)CO₂R² group wherein R¹ is a hydrogen atom and the like, and R² is a C₁₋₆ alkyl group, etc., a -C(R³)=NOR⁴ group wherein R³ is a hydrogen atom and the like, and R⁴ is a C₁₋₆ alkyl group, etc., or the like, Y is a halogen atom or the like, and Q is CH or a nitrogen atom, or a salt thereof.

## Description

### [Technical Field]

The present invention relates to azole compounds having a dithiolane ring and salts thereof, medical antifungal agents containing said compounds as active ingredients, and uses thereof.

### [Background Art]

It has been known that azole compounds having a dithiolane ring exhibit antifungal activity and are useful as agricultural fungicides or wood preservatives (see, for example, Patent Literatures 1, 4 and 5). They are also known to be useful as medical antifungal agents (see, for example, Patent Literatures 2 and 3), and some compounds are commercially available under the generic names Lanoconazole and Luliconazole. These prior art documents disclose several compounds in which the dithiolane ring is substituted with a pyridyl group, but do not disclose any compounds having the substituents that the compounds of the present invention have, nor disclose their use as medical antifungal agents at all.

### [Citation List]

### [Patent Literature]

[Patent Literature 1]
   JP Patent Publication No. S61-57565
[Patent Literature 2]
   JP Patent No. 1540657
[Patent Literature 3]
   JP Patent No. 3278738
[Patent Literature 4]
   JP Patent Publication No. 2000-26214
[Patent Literature 5]
   WO 2016/016118

### [Summary of Invention]

### [Technical Problem]

These conventional techniques did not have sufficient therapeutic activity against fungal infections such as localized fungal infections and systemic fungal infections and the like, caused by fungi such as Trichophyton, Candida, Aspergillus and the like. In addition, drugs having a dithiolane ring, such as Luliconazole and the like, have a problem that they cannot fully exert therapeutic activity against fungal infections, and are particularly ineffective against Candida. Therefore, there has been a demand for the development of medical antifungal agents that exhibit superior antifungal activity.

### [Solution to Problem]

The present inventors have conducted intensive studies in an attempt to solve the above-mentioned problems and found that the azole compounds in which the dithiolane ring has a pyridine ring with a specific substituent have superior antifungal activity against fungi such as Trichophyton, Candida, Aspergillus and the like, particularly Candida fungi, which resulted in the completion of the present invention.

That is, the present invention relates to at least the following inventions.
[1] A compound represented by the general formula (I) 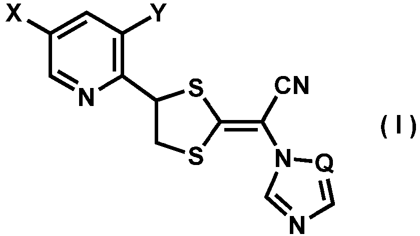 wherein
   X is a halogen atom, a cyano group, an amino group, a hydroxy group, a formyl group, a cyano C₁₋₆ alkyl group, a hydroxy C₁₋₆ alkyl group, a halo C₁₋₆ alkyl group, a C₂₋₆ alkenyl group, a C₂₋₆ alkynyl group, a C₁₋₆ alkoxy group, a halo C₁₋₆ alkoxy group, a C₂₋₆ alkenyloxy group, a C₂₋₆ alkynyloxy group, a C₁₋₆ alkoxy C₁₋₆ alkoxy group, a cyano C₁₋₆ alkoxy group, a C₁₋₆ alkyl-carbonyl group, a mono (C₁₋₆ alkyl-carbonyl) amino group, a mono (halo C₁₋₆ alkyl-carbonyl) amino group, a mono (phenyl C₁₋₆ alkyl-carbonyl) amino group, a tetrahydro-2H-pyran-2-yloxy group, a tetrahydro-2H-pyran-2-yloxymethyl group, a dioxolanyl group, a substituted dioxolanyl group having 1 to 5 substituents each independently selected from substituent group Z, a 2-oxo-1,3-dioxolan-4-yl group, an isoxazolinyl group, a substituted isoxazolinyl group having 1 to 4 substituents each independently selected from substituent group Z, a -N(R¹)CO₂R² group wherein R¹ is a hydrogen atom or a C₁₋₆ alkyl group, and R² is a C₁₋₆ alkyl group, a cyano C₁₋₆ alkyl group, a C₃₋₆ cycloalkyl group, a C₁₋₆ haloalkyl group, a C₁₋₆ alkoxy C₁₋₆ alkyl group, a C₃₋₆ cycloalkyl C₁₋₆ alkyl group, a C₁₋₆ alkoxycarbonylamino C₃₋₆ cycloalkyl C₁₋₆ alkyl group, a C₁₋₆ alkyl C₃₋₆ cycloalkyl C₁₋₆ alkyl group, a C₂₋₆ alkynyl C₃₋₆ cycloalkyl C₁₋₆ alkyl group, a cyano C₃₋₆ cycloalkyl C₁₋₆ alkyl group, an oxo C₃₋₆ cycloalkyl C₁₋₆ alkyl group, an oxotetrahydrofuranyl C₁₋₆ alkyl group, a halo C₃₋₆ cycloalkyl C₁₋₆ alkyl group, an oxetanyl C₁₋₆ alkyl group, a (3-oxabicyclo[3.1.0]hexan-6-yl)methyl group, a pyridyl C₁₋₆ alkyl group, a substituted pyridyl C₁₋₆ alkyl group having, on a ring, 1 to 4 substituents each independently selected from substituent group Z, a pyrazolyl C₁₋₆ alkyl group, a substituted pyrazolyl C₁₋₆ alkyl group having, on a ring, 1 to 3 substituents each independently selected from substituent group Z, a phenyl C₃₋₆ cycloalkyl C₁₋₆ alkyl group, a phenyl C₁₋₆ alkyl group, or a substituted phenyl C₁₋₆ alkyl group having, on a ring, 1 to 5 substituents each independently selected from substituent group Z, or a -C(R³)=NOR⁴ group wherein R³ is a hydrogen atom, an amino group or a C₁₋₆ alkyl group, and R⁴ is a hydrogen atom, a C₁₋₆ alkyl group, a C₃₋₆ cycloalkyl C₁₋₆ alkyl group, a halo C₃₋₆ cycloalkyl C₁₋₆ alkyl group, a phenyl C₁₋₆ alkyl group, or a substituted phenyl C₁₋₆ alkyl group having, on a ring, 1 to 5 substituents each independently selected from substituent group Z,
   Y is a hydrogen atom, a halogen atom, a cyano group, or a C₁₋₆ alkyl group,
   Q is CH or a nitrogen atom, and
   substituent group Z comprises a halogen atom, a nitro group, a cyano group, a C₁₋₆ alkyl group, a halo C₁₋₆ alkyl group, a C₁₋₆ alkoxy group, a halo C₁₋₆ alkoxy group, a C₁₋₆ alkylsulfanyl group, a C₁₋₆ alkylsulfinyl group, a C₁₋₆ alkylsulfonyl group, a mono (C₁₋₆ alkyl-carbonyl) amino group, a C₁₋₆ alkoxy-carbonyl group, a C₁₋₆ alkyl-carbonyl group, and a mono(C₁₋₆ alkyl)aminocarbonyl group, or a salt thereof.
[2] The compound or salt thereof of [1], wherein
   X is a -N(R¹)CO₂R² group wherein R¹ is a hydrogen atom or a C₁₋₆ alkyl group, and R² is a C₁₋₆ alkyl group, a C₃₋₆ cycloalkyl group, a C₃₋₆ cycloalkyl C₁₋₆ alkyl group, a halo C₃₋₆ cycloalkyl C₁₋₆ alkyl group, a phenyl C₁₋₆ alkyl group, or a substituted phenyl C₁₋₆ alkyl group having, on a ring, 1 to 5 substituents each independently selected from substituent group Z (substituent group Z is as defined in claim 1), or a -C(R³)=NOR⁴ group wherein R³ is a hydrogen atom, an amino group or a C₁₋₆ alkyl group, and R⁴ is a C₁₋₆ alkyl group, a C₃₋₆ cycloalkyl C₁₋₆ alkyl group, a halo C₃₋₆ cycloalkyl C₁₋₆ alkyl group, a phenyl C₁₋₆ alkyl group, or a substituted phenyl C₁₋₆ alkyl group having, on a ring, 1 to 5 substituents each independently selected from substituent group Z (substituent group Z is as defined in claim 1), and Y is a halogen atom.
[3] The compound or salt thereof of [1], wherein
   X is a -N(R¹)CO₂R² group wherein R¹ is a hydrogen atom, and R² is a C₁₋₆ alkyl group, a C₃₋₆ cycloalkyl C₁₋₆ alkyl group, a halo C₃₋₆ cycloalkyl C₁₋₆ alkyl group, or a phenyl C₁₋₆ alkyl group, or a - C(R³)=NOR⁴ group wherein R³ is a hydrogen atom, and R⁴ is a C₁₋₆ alkyl group, a C₃₋₆ cycloalkyl C₁₋₆ alkyl group, a halo C₃₋₆ cycloalkyl C₁₋₆ alkyl group, or a phenyl C₁₋₆ alkyl group,
   Y is a halogen atom, and
   Q is a nitrogen atom.
[4] A pharmaceutical composition comprising the compound or salt thereof of [1], and a pharmaceutically acceptable inert carrier or diluent.
[5] An antifungal agent comprising the compound or salt thereof of [1] as an active ingredient.
[6] A therapeutic agent for a fungal infection, comprising the compound or salt thereof of [1] as an active ingredient.
[7] A method for inhibiting fungal growth in a mammal, comprising administering to the mammal a pharmacologically effective amount of the compound or salt thereof of [1].
[8] A method for treating a fungal infection in a mammal, comprising administering to the mammal a pharmacologically effective amount of the compound or salt thereof of [1].
[9] The compound or salt thereof of [1], for use in inhibiting fungal growth.
[10] The compound or salt thereof of [1], for use in the treatment of a fungal infection.
[11] Use of the compound or salt thereof of [1], for the manufacture of an antifungal agent.
[12] Use of the compound or salt thereof of [1], for the manufacture of a therapeutic agent for a fungal infection.

### [Advantageous Effects of Invention]

According to the present invention, novel azole compounds having superior antifungal activity can be provided.

### [Description of Embodiments]

In the definition of the compound represented by the general formula (I) of the present invention, various groups are described in detail as follows. Unless otherwise specified, the description of each group also includes the case where it is a part of another group.

The "halogen atom" is a fluorine atom, a chlorine atom, a bromine atom or an iodine atom.

The "C₁₋₆ alkyl group" is a linear or branched alkyl group having 1 to 6 carbon atoms, and examples thereof include a methyl group, an ethyl group, a propyl group, a 2-propyl group, a butyl group, a 2-butyl group, a 2-methylpropyl group, a 1,1-dimethylethyl group, a pentyl group, a 3-pentyl group, a 3-methylbutyl group, a hexylgroup, a 3-hexylgroup, a 4-methylpentyl group and the like.

The "C₂₋₆ alkenyl group" is a linear or branched alkenyl group having 2 to 6 carbon atoms, and examples thereof include a vinyl group, a 2-propenyl group, a 2-butenyl group, a 3-butenyl group, a 2-methyl-1-propenyl group, a 1,1-dimethyl-2-propenyl group, a 3-pentenyl group, a 4-pentenyl group, a 3-hexenyl group, a 4,4-dimethyl-2-butenyl group and the like.

The "C₂₋₆ alkynyl group" is a linear or branched alkynyl group having 2 to 6 carbon atoms, and examples thereof include an ethynyl group, a 2-propynyl group, a 2-butynyl group, a 3-butynyl group, a 1-methyl-2-propynyl group, a 1,1-dimethyl-2-propynyl group, a 3-pentynyl group, a 4-pentynyl group, a 3-hexynyl group, a 2,2-dimethyl-3-butynyl group and the like.

The "C₁₋₆ alkoxy group" is a group in which a linear or branched alkyl group having 1 to 6 carbon atoms is bonded to an oxygen atom, and examples thereof include a methoxy group, an ethoxy group, a propoxy group, a 2-propoxy group, a butoxy group, a 2-butoxy group, a 2-methylpropoxy group, a 1,1-dimethylethoxy group, a pentyloxy group, a 3-pentyloxy group, a 3-methylbutoxy group, a hexyloxy group, a 3-hexyloxy group, a 4-methylpentyloxy group and the like.

The "C₂₋₆ alkenyloxy group" is a group in which a linear or branched alkenyl group having 2 to 6 carbon atoms is bonded to an oxygen atom, and examples thereof include a vinyloxy group, a 2-propenyloxy group, a 2-butenyloxy group, a 3-butenyloxy group, a 2-methyl-1-propenyloxy group, a 1,1-dimethyl-2-propenyloxy group, a 3-pentenyloxy group, a 4-pentenyloxy group, a 3-hexenyloxy group, a 4,4-dimethyl-2-butenyloxy group and the like.

The "C₂₋₆ alkynyloxy group" is a group in which a linear or branched alkynyl group having 2 to 6 carbon atoms is bonded to an oxygen atom, and examples thereof include a 2-propynyloxy group, a 2-butynyloxy group, a 3-butynyloxy group, a 1-methyl-2-propynyloxy group, a 1,1-dimethyl-2-propynyloxy group, a 3-pentynyloxy group, a 4-pentynyloxy group, a 3-hexynyloxy group, a 2,2-dimethyl-3-butynyloxy group and the like.

The "C₁₋₆ alkylsulfanyl group" is a group in which a linear or branched alkyl group having 1 to 6 carbon atoms is bonded to a sulfur atom, and examples thereof include a methylthio group, an ethylthio group, a propylthio group, a 2-propylthio group, a butylthio group, a 2-butylthio group, a 2-methylpropylthio group, a 1,1-dimethylethylthio group, a pentylthio group, a 3-pentylthio group, a 3-methylbutylthio group, a hexylthio group, a 3-hexylthio group, a 4-methylpentylthio group and the like.

The "C₁₋₆ alkylsulfinyl group" is a group in which the sulfur atom of a C₁₋₆ alkylsulfanyl group is oxidized to SO, and examples thereof include a methylsulfinyl group, an ethylsulfinyl group, a propylsulfinyl group, a 2-propylsulfinyl group, a butylsulfinyl group, a 2-butylsulfinyl group, a 2-methylpropylsulfinyl group, a 1,1-dimethylethylsulfinyl group, a pentylsulfinyl group, a 3-pentylsulfinyl group, a 3-methylbutylsulfinyl group, a hexylsulfinyl group, a 3-hexylsulfinyl group, a 4-methylpentylsulfinyl group and the like.

The "C₁₋₆ alkylsulfonyl group" is a group in which the sulfur atom of a C₁₋₆ alkylsulfanyl group is oxidized to SO₂, and examples thereof include a methylsulfonyl group, an ethylsulfonyl group, a propylsulfonyl group, a 2-propylsulfonyl group, a butylsulfonyl group, a 2-butylsulfonyl group, a 2-methylpropylsulfonyl group, a 1,1-dimethylethylsulfonyl group, a pentylsulfonyl group, a 3-pentylsulfonyl group, a 3-methylbutylsulfonyl group, a hexylsulfonyl group, a 3-hexylsulfonyl group, a 4-methylpentylsulfonyl group and the like.

The "C₃₋₆ cycloalkyl group" is a cycloalkyl group having 3 to 6 carbon atoms, and examples thereof include a cyclopropyl group, a 1-methylcyclopropyl group, a 2-methylcyclopropyl group, a cyclobutyl group, a cyclopentyl group, a cyclohexyl group and the like.

The "C₃₋₆ cycloalkoxy group is a cycloalkoxy group having 3 to 6 carbon atoms, and examples thereof include a cyclopropyloxy group, a cyclopentyloxy group, and a cyclohexyloxy group.

The "cyano C₁₋₆ alkyl group" is an alkyl group having 1 to 6 carbon atoms and having one cyano group at substitutable position, and examples thereof include a cyanomethyl group, a 1-cyanoethyl group, a 2-cyanoethyl group, a 1-cyanopropyl group, a 3-cyanopropyl group, a 2-cyanopropan-2-yl group, a 1-cyanobutyl group, a 4-cyanobutyl group, a 5-cyanopentyl group, a 6-cyanohexylgroup and the like.

The "cyano C₃₋₆ cycloalkyl group" is a cycloalkyl group having 3 to 6 carbon atoms and having one cyano group at substitutable position, and examples thereof include a 1-cyanocyclopropyl group, a 2-cyanocyclopropyl group, a 1-cyanocyclobutyl group, a 3-cyanocyclobutyl group, a 1-cyanocyclopentyl group and the like.

The "hydroxy C₁₋₆ alkyl group" is an alkyl group having 1 to 6 carbon atoms and having one hydroxy group at substitutable position, and examples thereof include a hydroxymethyl group, a 1-hydroxyethyl group, a 2-hydroxyethyl group, a 1-hydroxypropyl group, a 2-hydroxypropyl group, a 3-hydroxypropyl group, a 1-hydroxybutyl group, a 4-hydroxybutyl group and the like.

The "C₁₋₆ alkyl-carbonyl group" is a group in which an alkyl group having 1 to 6 carbon atoms is bonded to carbonyl (-CO-), and examples thereof include an acetyl group, a propionyl group, a 2-methylpropionyl group, a 2,2-dimethylpropionyl group, a butanoyl group, a 2-methylbutanoyl group, a 3-methylbutanoyl group, a 2-ethylbutanoyl group, a 2,2-dimethylbutanoyl group, a 2,3-dimethylbutanoyl group, a 3,3-dimethylbutanoyl group, a pentanoyl group, a 2-methylpentanoyl group, a 3-methylpentanoyl group, a 4-methylpentanoyl group, a hexanoyl group and the like.

Examples of the "mono(C₁₋₆ alkyl-carbonyl) amino group" include a methylcarbonylamino group, an ethylcarbonylamino group, a n-propylcarbonylamino group, or a 2-propylcarbonylamino group, a tertiary-butoxycarbonylamino group and the like.

The "C₁₋₆ alkoxy-carbonyl group" is a group in which an alkoxy group having 1 to 6 carbon atoms is bonded to carbonyl (-CO-), and examples thereof include a methoxycarbonyl group, an ethoxycarbonyl group, a propoxycarbonyl group, a 2-propoxycarbonyl group, a butoxycarbonyl group, a 2-butoxycarbonyl group, a 2-methylpropoxycarbonyl group, a pentyloxycarbonyl group, a 3-pentyloxycarbonyl group, a 3-methylbutoxycarbonyl group and the like.

The expressions of "C₁₋₆", "C₂₋₆" and the like indicate the range of carbon numbers of various substituents. Furthermore, the above-mentioned definition can also be applied to the groups to which the above-mentioned substituents are bonded. For example, a "halo C₁₋₆ alkyl group" refers to a linear or branched alkyl group having 1 to 6 carbon atoms to which a halogen atom is bonded. In addition, a "phenyl C₁₋₆ alkyl group" refers to a group in which a linear or branched alkyl group having 1 to 6 carbon atoms is bonded to a phenyl group, and examples thereof include a benzyl group, a phenethyl group, an α-methylbenzyl group and the like.

Examples of the salt of the compound represented by the general formula (I) of the present invention include acid addition salts to the nitrogen atoms of the pyridine ring, the imidazole ring and the triazole ring, each having basicity. Examples of the corresponding acid include inorganic acids such as hydrochloric acid, sulfuric acid, nitric acid and the like, and organic acids such as formic acid, acetic acid, trifluoroacetic acid, methanesulfonic acid, trifluoromethanesulfonic acid, p-toluenesulfonic acid and the like. The compound represented by the general formula (I) and salts thereof of the present invention may contain one or plural number of asymmetric centers in the structural formula, and in some cases, two or more optical isomers and diastereomers may be present. The present invention encompasses any optical isomers and mixtures containing them at any ratio. In addition, the compound represented by the general formula (I) and salts thereof of the present invention may have two or more geometric isomers derived from a C-C double bond or a C-N double bond in the structural formula. The present invention encompasses any geometric isomers and the mixtures containing them at any ratio.

The compound represented by the general formula (I) or a salt thereof of the present invention
is preferably the compound or salt thereof wherein X is a - N(R¹)CO₂R² group wherein R¹ is a hydrogen atom or a C₁₋₆ alkyl group, and R² is a C₁₋₆ alkyl group, a C₃₋₆ cycloalkyl group, a C₃₋₆ cycloalkyl C₁₋₆ alkyl group, a halo C₃₋₆ cycloalkyl C₁₋₆ alkyl group, a phenyl C₁₋₆ alkyl group, or a substituted phenyl C₁₋₆ alkyl group having, on a ring, 1 to 5 substituents each independently selected from substituent group Z (substituent group Z is as defined above), or a -C(R³)=NOR⁴ group wherein R³ is a hydrogen atom, an amino group or a C₁₋₆ alkyl group, and R⁴ is a C₁₋₆ alkyl group, a C₃₋₆ cycloalkyl C₁₋₆ alkyl group, a halo C₃₋₆ cycloalkyl C₁₋₆ alkyl group, a phenyl C₁₋₆ alkyl group, or a substituted phenyl C₁₋₆ alkyl group having, on a ring, 1 to 5 substituents each independently selected from substituent group Z (substituent group Z is as defined above), Y is a halogen atom, and Q is CH or a nitrogen atom,
more preferably the compound or salt thereof wherein X is a - N(R¹)CO₂R² group wherein R¹ is a hydrogen atom, and R² is a C₁₋₆ alkyl group, a C₃₋₆ cycloalkyl C₁₋₆ alkyl group, a halo C₃₋₆ cycloalkyl C₁₋₆ alkyl group, or a phenyl C₁₋₆ alkyl group, or a - C(R³)=NOR⁴ group wherein R³ is a hydrogen atom, and R⁴ is a C₁₋₆ alkyl group, a C₃₋₆ cycloalkyl C₁₋₆ alkyl group, a halo C₃₋₆ cycloalkyl C₁₋₆ alkyl group, or a phenyl C₁₋₆ alkyl group, Y is a halogen atom, and Q is a nitrogen atom.

The compound represented by the general formula (I) of the present invention can be produced by the following Production Methods 1 to 4.

### Production Method 1

The compound of the general formula (I) in which X is Xa, i.e., a compound represented by the general formula (I-1), can be produced by the method shown below. 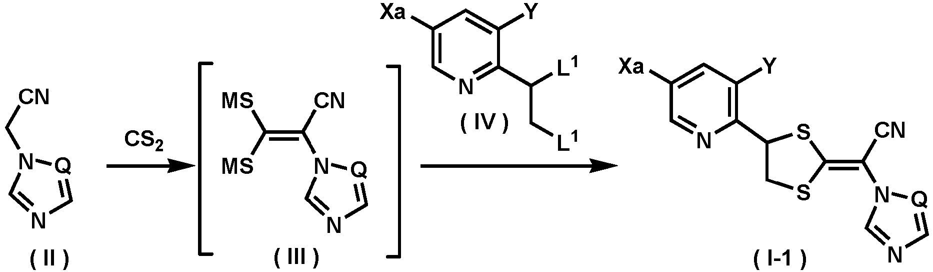 wherein Y and Q are as defined above, Xa is a halogen atom, a cyano group, a halo C₁₋₆ alkyl group, a C₂₋₆ alkenyl group, a C₂₋₆ alkynyl group, a C₁₋₆ alkoxy group, a halo C₁₋₆ alkoxy group, a C₂₋₆ alkenyloxy group, a C₂₋₆ alkynyloxy group, a dioxolanyl group, a substituted dioxolanyl group having 1 to 5 substituents each independently selected from substituent group Z (substituent group Z is as defined above), an isoxazolinyl group, or a substituted isoxazolinyl group having 1 to 4 substituents each independently selected from substituent group Z (substituent group Z is as defined above), L¹ are the same or different and each is a leaving group such as a methanesulfonyloxy group, a trifluoromethanesulfonyloxy group, a p-toluenesulfonyloxy group, a benzenesulfonyloxy group, a chlorine atom, a bromine atom, an iodine atom or the like, and M is an alkali metal atom such as a lithium atom, a sodium atom, a potassium atom or the like.

That is, the target compound represented by the general formula (I-1) can be produced in the same manner as in the production method described in JP-A-61-57565, by reacting a compound represented by the general formula (II) with carbon disulfide in the presence of a base and an inert solvent to convert the compound into a corresponding dithiolate salt (III), and then reacting the salt, with or without isolation, with a compound represented by general formula (IV).

The inert solvent that can be used in this reaction is not limited as long as it does not inhibit the progress of this reaction. For example, alcohols such as methanol, ethanol, isopropyl alcohol and the like, polar solvents such as dimethyl sulfoxide, dimethylformamide, 1,3-dimethyl-2-imidazolidinone, acetonitrile and the like, water or a mixture of these solvents can be used. The amount of the inert solvent to be used is not limited as long as the reaction proceeds, and may be appropriately selected from the range of 0.5 to 50 L per 1 mol of the compound represented by the general formula (II) or the compound represented by the general formula (IV). Examples of the base include sodium carbonate, potassium carbonate, sodium hydroxide, potassium hydroxide and the like. These can be used as a solid or a solution dissolved in an inert solvent. The amount of the base to be used may be selected from the range of 2 to 8-fold mol per 1 mol of the compound represented by the general formula (II), preferably in the range of 4 to 6-fold mol.

Since this reaction is an equimolar reaction, the compound represented by the general formula (II), carbon disulfide and the compound represented by the general formula (IV) may be used in equimolar amounts, but any of them may be used in an excess amount. The reaction temperature may be selected from the range of 0 to 100°C, and is preferably around room temperature. The reaction time varies depending on the reaction scale and reaction temperature, and can be selected from the range of 0.5 to 24 hr. The obtained compound may be a mixture of geometric isomers (E) and (Z). In this case, each isomer can be isolated and purified by a method such as silica gel column chromatography, recrystallization or the like. As the recrystallization solvent, ethanol, ethyl acetate, ether, hexane, acetone or the like, or a mixture of these solvents can be used, but the solvent is not particularly limited.

### Production Method 2

The compound of the general formula (I) in which X is a - N(R¹)CO₂R² group wherein R¹ and R² are as defined above, i.e., a compound represented by the general formula (I-2), can be produced by the method shown below. 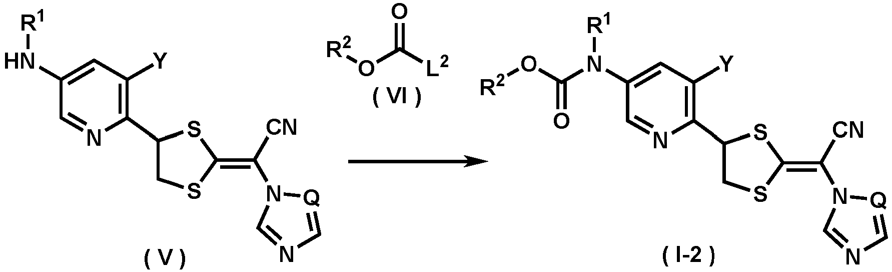 wherein Y, R¹, R² and Q are as defined above, and L² is a leaving group such as a halogen atom or the like.

That is, a compound represented by the general formula (I-2) can be produced by reacting a compound represented by the general formula (V) with a compound represented by the general formula (VI) in the presence of a base and an inert solvent.

A compound of the general formula (V) in which R¹ is a hydrogen atom is included in the compound represented by the general formula (I).

Examples of the base that can be used in this reaction include inorganic bases such as sodium carbonate, potassium carbonate, sodium hydroxide, potassium hydroxide and the like, and organic bases such as triethylamine, diisopropylethylamine, pyridine, dimethylaminopyridine and the like. Organic bases can also be used as solvents. The amount of the base to be used is may be selected from the range of 0.8 to 8-fold mol per 1 mol of the compound represented by the general formula (V), preferably in the range of 1 to 3-fold mol.

The inert solvent that can be used in this reaction is not limited as long as it does not inhibit the progress of this reaction. For example, alcohols such as methanol, ethanol, isopropyl alcohol and the like, polar solvents such as dimethyl sulfoxide, dimethylformamide, 1,3-dimethyl-2-imidazolidinone, acetonitrile and the like, chain or cyclic ethers such as diethyl ether, methyl tertiary-butyl ether, ethyl tertiary-butyl ether, dioxane, tetrahydrofuran, 1,2-dimethoxyethane and the like, or a mixture of these solvents can be used. The amount of the inert solvent to be used is not limited as long as the reaction proceeds, and may be appropriately selected from the range of 0.5 to 50 L per 1 mol of the compound represented by the general formula (V)

The reaction temperature may be appropriately selected from the range of -20°C and the boiling point of the inert solvent, preferably in the range of 0°C to 50°C. The reaction time varies depending on the reaction scale and reaction temperature, and can be selected from the range of 0.5 to 24 hr. Since this reaction is an equimolar reaction, the compound represented by the general formula (V) and the compound represented by the general formula (VI) may be used in equimolar amounts, but either of the reactants may be used in excess. After completion of the reaction, the target product can be obtained by isolation according to a conventional method, and, if necessary, purification by recrystallization, column chromatography or the like.

The compound of the general formula (I) in which X is - C(R³)=NOR⁴ group wherein R³ and R⁴ are as defined above, i.e., a compound represented by the general formula (I-3), can be produced by the method described in Production Method 3 or Production Method 4.

### Production Method 3

The compound of the general formula (I-3) in which R³ is R^{3a}, i.e., a compound represented by the general formula (I-3a), can be produced by the method shown below. 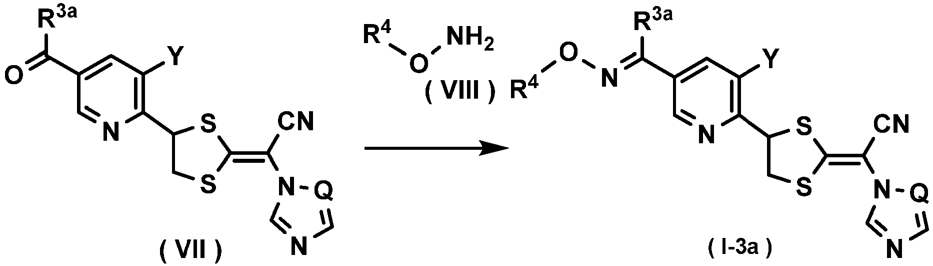 wherein Y, R⁴ and Q are as defined above, and R^{3a} is a hydrogen atom or a C₁₋₆ alkyl group.

That is, a compound represented by the general formula (I-3a) can be produced by reacting a compound represented by the general formula (VII) with a compound represented by the general formula (VIII) in an inert solvent.

A compound represented by the general formula (VII) is included in the compound represented by the general formula (I).

The inert solvent that can be used in this reaction is not limited as long as it does not inhibit the progress of this reaction. For example, alcohols such as methanol, ethanol, isopropyl alcohol and the like, polar solvents such as dimethyl sulfoxide, dimethylformamide, 1,3-dimethyl-2-imidazolidinone, acetonitrile and the like, chain or cyclic ethers such as diethyl ether, methyl tertiary-butyl ether, ethyl tertiary-butyl ether, dioxane, tetrahydrofuran, 1,2-dimethoxyethane and the like, or a mixture of these solvents can be used. The amount of the inert solvent to be used is not limited as long as the reaction proceeds, and may be appropriately selected from the range of 0.5 to 50 L per 1 mol of the compound represented by the general formula (VII).

The reaction temperature may be appropriately selected from the range of -20°C and the boiling point of the inert solvent, preferably in the range of 0°C to 50°C. The reaction time varies depending on the reaction scale and reaction temperature, and can be selected from the range of 0.5 to 24 hr. Since this reaction is an equimolar reaction, the compound represented by the general formula (VII) and the compound represented by the general formula (VIII) may be used in equimolar amounts, but either of the reactants may be used in excess. The compound represented by the general formula (VIII) may be used in the form of an acid salt such as a hydrochloride, a sulfurate or the like. After completion of the reaction, the target product can be obtained by isolation according to a conventional method, and, if necessary, purification by recrystallization, column chromatography or the like.

### Production Method 4

The compound of the general formula (I-3) in which R³ is R^{3b}, i.e., a compound represented by the general formula (I-3b), can be produced by the method shown below. 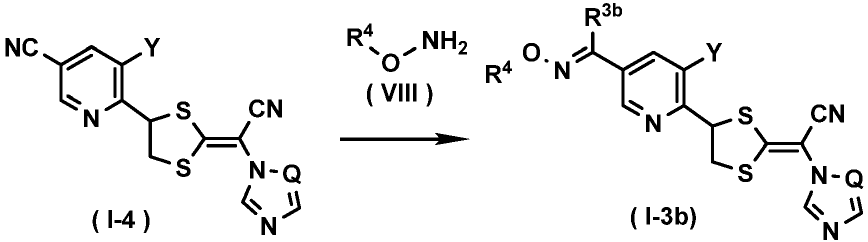 wherein Y, R⁴ and Q are as defined above, and R^{3b} is an amino group.

That is, a compound represented by the general formula (I-3b) can be produced by reacting a compound represented by the general formula (I-4) with a compound represented by the general formula (VIII) in an inert solvent. The production conditions for this step are the same as those for Production Method 3.

### Production Method 1 of raw material intermediate

The aforementioned raw material intermediate represented by the general formula (IV) can be produced by the method shown below. 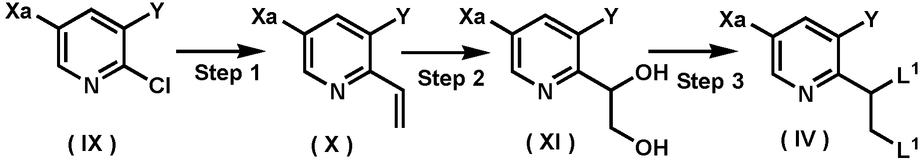 wherein Xa, Y and L¹ are as defined above.

That is, the raw material intermediate represented by the general formula (IV) can be produced from a compound represented by the general formula (IX) by the above Steps 1 to 3.

### <Step 1>

A compound represented by the general formula (X) can be produced by subjecting a compound represented by the general formula (IX) to a coupling reaction with a vinylating reagent in the presence of a metal catalyst and a base, in an inert solvent.

Examples of the vinylating reagent used in this reaction include Grignard reagents such as vinylmagnesium bromide and the like, boron compounds such as vinylboric acid, potassium vinyltrifluoroborate and the like, tin compounds such as tributyl(vinyl)tin and the like, and the like.

The metal catalyst that can be used in this reaction is preferably a palladium catalyst, and examples thereof include palladium acetate, palladium chloride, tetrakistriphenylphosphine palladium(0), [1,1'-bis(diphenylphosphino)ferrocene]palladium(II) dichloride acetone adduct and the like. If necessary, a ligand such as triphenylphosphine, tri(tertiary-butyl)phosphine, 1,2-bisdiphenylphosphinoethane, 1,3-bisdiphenylphosphinopropane, 1,4-bisdiphenylphosphinobutane and the like may be added. The amount of the palladium catalyst to be used may be appropriately selected from the range of 0.0001-fold mol to 0.2-fold mol per 1 mol of the compound represented by the general formula (IX). The amount of the ligand to be used may be appropriately selected from the range of 0.5-fold mol to 10-fold mol per 1 mol of the palladium catalyst.

Examples of the base that can be used in this reaction include inorganic bases such as sodium carbonate, potassium carbonate, sodium hydroxide, potassium hydroxide and the like, and organic bases such as triethylamine, diisopropylethylamine, pyridine, dimethylaminopyridine and the like. Organic bases can also be used as solvents. The amount of the base to be used may be selected from the range of 0.8 to 8-fold mol per 1 mol of the compound represented by the general formula (IX), preferably in the range of 1 to 3-fold mol.

The inert solvent that can be used in this reaction is not limited as long as it does not inhibit the progress of this reaction. For example, polar solvents such as dimethyl sulfoxide, dimethylformamide, 1,3-dimethyl-2-imidazolidinone, acetonitrile and the like, chain or cyclic ethers such as diethyl ether, methyl tertiary-butyl ether, ethyl tertiary-butyl ether, dioxane, tetrahydrofuran, 1,2-dimethoxyethane and the like, or a mixture of these solvents can be used. The amount of the inert solvent to be used is not limited as long as the reaction proceeds, and may be appropriately selected from the range of 0.5 to 50 L per 1 mol of the compound represented by the general formula (IX).

The reaction temperature may be appropriately selected from the range of -20°C and the boiling point of the inert solvent, preferably in the range of 0°C to 50°C. The reaction time varies depending on the reaction scale and reaction temperature, and can be selected from the range of 0.5 to 24 hr. Since this reaction is an equimolar reaction, the compound represented by the general formula (IX) and the vinylating reagent may be used in equimolar amounts, but either of the reactants may be used in excess. After completion of the reaction, the target product can be obtained by isolation according to a conventional method, and, if necessary, purification by recrystallization, column chromatography or the like. Alternatively, the target product can also be used in the next step without isolation.

### <Step 2>

A compound represented by the general formula (XI) can be produced by reacting a compound represented by the general formula (X) with an oxidant in an inert solvent.

Examples of the oxidant that can be used in this reaction include permanganates, chromates, hydrogen peroxide, periodates, osmium tetroxide and the like. Among them, oxidation with 4-methylmorpholine-N-oxide using osmium tetroxide as a catalyst is particularly preferred. The amount of the oxidant to be used may be appropriately selected from the range of 0.8-fold mol to 4-fold mol per 1 mol of the compound represented by the general formula (X), preferably in the range of 1-fold mol to 2-fold mol.

The inert solvent that can be used in this reaction is not limited as long as it does not inhibit the progress of this reaction. For example, alcohols such as methanol, ethanol, isopropyl alcohol and the like, chain or cyclic ethers such as diethyl ether, methyl tertiary-butyl ether, ethyl tertiary-butyl ether, dioxane, tetrahydrofuran, 1,2-dimethoxyethane and the like, ketones such as acetone, methyl ethyl ketone and the like, polar solvents such as dimethyl sulfoxide, dimethylformamide, 1,3-dimethyl-2-imidazolidinone, acetonitrile and the like, aromatic hydrocarbons such as toluene, chlorobenzene, fluorobenzene, dichlorobenzene, difluorobenzene and the like, dichloromethane, chloroform, carbon tetrachloride, dichloroethane, water and the like, or a mixture of these solvents can be used. The amount of the inert solvent to be used is not limited as long as the reaction proceeds, and may be appropriately selected from the range of 0.5 to 50 L per 1 mol of the compound represented by the general formula (X).

The reaction temperature may be appropriately selected from the range of -20°C and the boiling point of the inert solvent, preferably in the range of 0°C to 50°C. The reaction time varies depending on the reaction scale and reaction temperature, and can be selected from the range of 0.5 to 24 hr. After completion of the reaction, the target product can be obtained by isolation according to a conventional method, and, if necessary, purification by recrystallization, column chromatography or the like. Alternatively, the target product can also be used in the next step without isolation.

### <Step 3>

The raw material intermediate represented by the general formula (IV) can be produced by reacting a compound represented by the general formula (XI) with a halogenating agent or a sulfonylating agent or the like in the presence of a base, in an inert solvent.

Examples of the halogenating agent that can be used in this reaction include carbonyl halide compounds such as oxalyl chloride, phosgene, diphosgene, triphosgene and the like, sulfur halide compounds such as thionyl chloride, thionyl bromide and the like, phosphorus halide compounds such as phosphorus oxychloride, phosphorus oxybromide, phosphorus trichloride, phosphorus tribromide, phosphorus pentachloride and the like, and the like. These halogenating agents may be used alone or in a mixture of two or more kinds thereof. The amount of the halogenating agent to be used, may be appropriately selected from the range of 0.5 to 10-fold mol relative to the compound represented by the general formula (XI). Examples of the sulfonylating agent include methanesulfonyl chloride, trifluoromethanesulfonyl chloride, p-toluenesulfonyl chloride and the like. The amount of the sulfonylating agent to be used may be appropriately selected from the range of 0.5 to 10-fold mol relative to the compound represented by the general formula (XI).

The inert solvent that can be used in this reaction is not limited as long as it does not markedly inhibit the progress of this reaction. Examples of the inert solvent include aromatic hydrocarbons such as benzene, toluene, xylene and the like, halogenated hydrocarbons such as methylene chloride, chloroform, carbon tetrachloride and the like, halogenated aromatic hydrocarbons such as chlorobenzene, dichlorobenzene and the like, and the like. These inert solvents may be used alone or in a mixture of two or more kinds thereof. The amount of the inert solvent to be used is not limited as long as it can dissolve the compound represented by the general formula (XI), and may be appropriately selected from the range of 0.5 to 50 L per 1 mol of the compound represented by the general formula (XI).

The reaction temperature may be selected from the range of about -20 to 150°C. The reaction time varies depending on the reaction scale, the reaction temperature and the like, and may be appropriately selected from the range of several minutes to 48 hr. After completion of the reaction, the raw material intermediate represented by the general formula (IV) can be obtained by isolation according to a conventional method, and, if necessary, purification by a recrystallization method, a distillation method, a column chromatography method or the like. Alternatively, the raw material intermediate may be used in the next step without isolation.

### Production Method 2 of raw material intermediate

The aforementioned raw material intermediate represented by the general formula (V) can be produced by the method shown below. 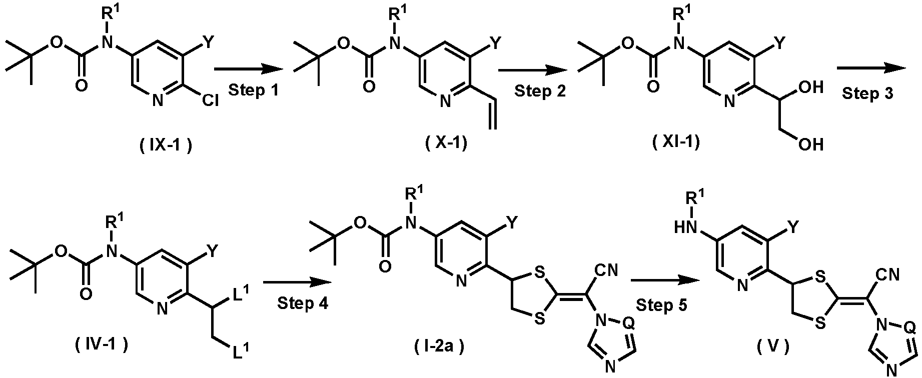 wherein R¹, Y, Q and L¹ are the same as above.

A compound represented by the general formula (IX-1) can be produced by subjecting a commercially available, corresponding 6-chloropyridine-3-carboxylate ester to hydrolysis, followed by a rearrangement reaction. The raw material intermediate represented by the general formula (V) can be produced from the compound represented by the general formula (IX-1) by the above Step 1 to 5. The production conditions for Steps 1 to 3 are the same as those for Steps 1 to 3 in Production Method 1 of raw material intermediate, and the production conditions for Step 4 are the same as those for Production Method 1.

### <Step 5>

The compound represented by the general formula (V) can be produced by subjecting a compound represented by the general formula (I-2a) to hydrolysis in the presence of an acid and in the presence or absence of an inert solvent.

Examples of the acid that can be used in this reaction include inorganic acids such as hydrochloric acid, sulfuric acid, nitric acid and the like, organic acids such as formic acid, acetic acid, propionic acid, trifluoroacetic acid, benzoic acid and the like, sulfonic acids such as methanesulfonic acid, trifluoromethanesulfonic acid, p-toluenesulfonic acid and the like, phosphoric acid and the like. The amount thereof to be used may be appropriately selected from the range of 0.01-fold mol to 10-fold mol relative to the compound represented by the general formula (I-2a). Acids can also be used as a solvent.

The inert solvent that can be used in this reaction is not limited as long as it does not markedly inhibit the progress of this reaction. Examples of the inert solvent include alcohols such as methanol, ethanol, isopropyl alcohol and the like, aromatic hydrocarbons such as benzene, toluene, xylene and the like, halogenated hydrocarbons such as methylene chloride, chloroform, carbon tetrachloride and the like, halogenated aromatic hydrocarbons such as chlorobenzene, dichlorobenzene and the like, chain or cyclic ethers such as diethyl ether, methyl tertiary-butyl ether, ethyl tertiary-butyl ether, dioxane, tetrahydrofuran, 1,2-dimethoxyethane and the like, esters such as ethyl acetate and the like, amides such as N,N-dimethylformamide, N,N-dimethylacetamide and the like, ketones such as acetone, methyl ethyl ketone and the like, polar solvents such as dimethyl sulfoxide, 1,3-dimethyl-2-imidazolidinone and the like, water and the like. These inert solvents may be used alone or in a mixture of two or more kinds thereof. The amount of the inert solvent to be used is not limited as long as it can dissolve the reaction reagents, and may be appropriately selected from the range of 0.5 L to 100 L per 1 mol of the compound represented by the general formula (I-2a). When an acid is used as a solvent, no solvent may be used.

The reaction temperature may be in the range of room temperature to the boiling point of the acid or inert solvent to be used. The reaction time varies depending on the reaction scale and reaction temperature, and may be appropriately selected from the range of several minutes to 48 hr. After completion of the reaction, the target product can be obtained by isolation from the reaction system containing it according to a conventional method, and, if necessary, purification by recrystallization, column chromatography or the like. The target product can also be isolated as an acid salt of the acid used. Alternatively, the target product can be used in the next step without purification.

### Production Method 3 of raw material intermediate

The aforementioned raw material intermediate represented by the general formula (VII) can be produced by the method shown below.

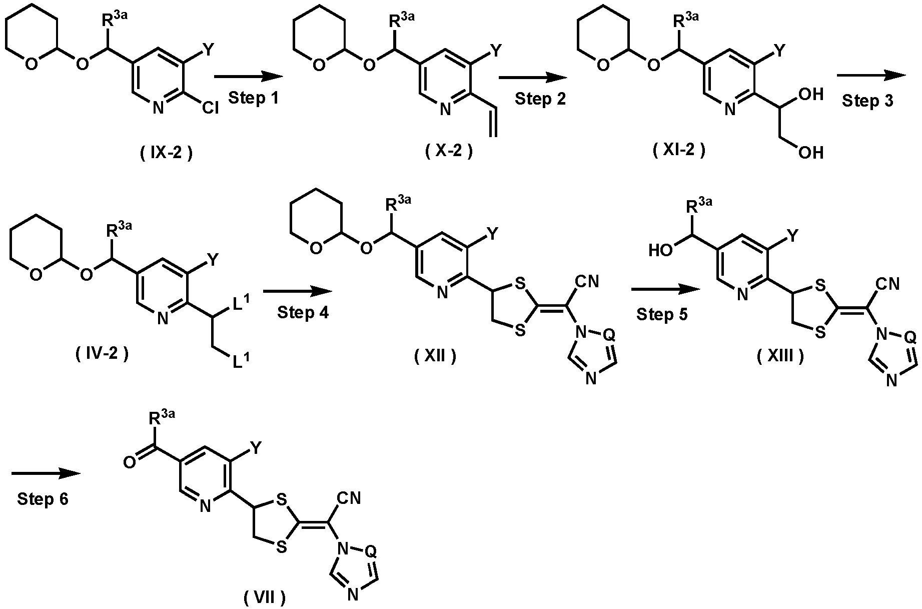

The raw material intermediate represented by the general formula (VII) can be produced from a compound represented by the general formula (IX-2), which is produced from a commercially available raw material by a known method, by the above Steps 1 to 6. The production conditions for Steps 1 to 3 are the same as those for Steps 1 to 3 in Production Method 1 of raw material intermediate, the production conditions for Step 4 are the same as those for Production Method 1, and the deprotection conditions for Step 5 are the same as those for Step 5 in Production Method 2 of raw material intermediate.

### <Step 6>

The compound represented by the general formula (VII) can be produced by reacting a compound represented by the general formula (XIII) with an oxidant in the presence of an inert solvent.

Examples of the oxidant that can be used in this reaction include permanganates, chromates, hydrogen peroxide, periodates, osmium tetroxide, oxalyl chloride/dimethyl sulfoxide (Swern oxidation), hypervalent iodine compounds (Dess-Martin oxidation) and the like. Among them, Swern oxidation is particularly preferred. The amount of the oxidant to be used may be appropriately selected from the range of 0.8-fold mol to 10-fold mol per 1 mol of the compound represented by the general formula (XIII), preferably in the range of 1-fold mol to 4-fold mol. Depending on the type of oxidant, a base may be required. Examples of the base that can be used include inorganic bases such as sodium carbonate, potassium carbonate, sodium hydroxide, potassium hydroxide and the like, and organic bases such as triethylamine, diisopropylethylamine, pyridine, dimethylaminopyridine and the like. Organic bases can also be used as solvents. The amount of the base to be used may be selected from the range of 2 to 8-fold mol per 1 mol of the compound represented by the general formula (XIII), preferably in the range of 3 to 5-fold mol.

The inert solvent that can be used in this reaction is not limited as long as it does not inhibit the progress of this reaction. For example, alcohols such as methanol, ethanol, isopropyl alcohol and the like, ketones such as acetone, methyl ethyl ketone and the like, polar solvents such as dimethyl sulfoxide, dimethylformamide, 1,3-dimethyl-2-imidazolidinone, acetonitrile and the like, aromatic hydrocarbons such as toluene, chlorobenzene, fluorobenzene, dichlorobenzene, difluorobenzene and the like, dichloromethane, chloroform, carbon tetrachloride, dichloroethane, water and the like, or a mixture of these solvents can be used. The amount of the inert solvent to be used is not limited as long as the reaction proceeds, and may be appropriately selected from the range of 0.5 to 50 L per 1 mol of the compound represented by the general formula (XIII).

The reaction temperature may be appropriately selected from the range of -20°C and the boiling point of the inert solvent, preferably in the range of 0°C to 50°C. The reaction time varies depending on the reaction scale and reaction temperature, and can be selected from the range of 0.5 to 24 hr. After completion of the reaction, the target product can be obtained by isolation according to a conventional method, and, if necessary, purification by recrystallization, column chromatography or the like. Alternatively, the target product can also be used in the next step without isolation.

Examples of the compounds of the present invention that can be produced in accordance with the above production examples are shown in Tables 1 to 3 and Tables 5 to 17, but the present invention is not limited to these. In the tables, "Me" indicates a methyl group, "Et" indicates an ethyl group, "Pr" indicates a propyl group, "Bu" indicates a butyl group, "Bn" indicates a benzyl group, "n-" indicates normal, "i-" indicates iso, "c-" indicates cyclo, and "t-" indicates tertiary. For those listed as NMR in the physical properties column in the tables, the ¹H-MNR data are shown in Table 4 and Tables 18 to Table 21. The "*" or "**" in the "Compound No." column indicates an optically active form on the dithiolane ring, with "*" indicating the R form and "**" indicating the S form, "d" indicates a diastereomer in which the substituent on the cyclopropane ring is a trans form, and ◆ indicates a hydrochloride. "•" indicates a bonding position.

### The general formula (I)

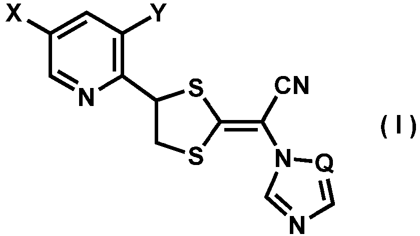

**[Table 1]**

| Compound No. | X | Y | Q | Physical property Melting point (°C) |
|---|---|---|---|---|
| 1 | Br | Cl | CH | 164-165 |
| 2 | NH₂ | Cl | CH | 178-179 |
| 3 | CH₂=CH | Cl | CH | 142-143 |
| 4 | NCCH₂O | Cl | CH | 162-163 |
| 5 | 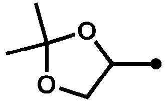 | Cl | CH | 171-172 |
| 6 | 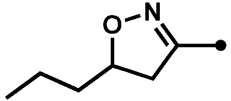 | Cl | CH | 133-134 |

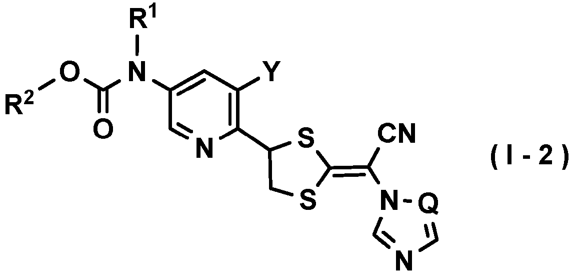

**[Table 2]**

| Compound No. | Y | R¹ | R² | Q | Physical property Melting point (°C) |
|---|---|---|---|---|---|
| 7 | Cl | H | Me | N | 214-215 |
| 8* | F | H | Et | N | 177-178 |
| 9* | Cl | H | Me | N | 146-147 |
| 10 | F | H | Et | N | 160-161 |
| 11 | Cl | H | Et | N | 185-186 |
| 12 | Cl | H | t-Bu | N | NMR |
| 13 | F | H | Bn | N | 168-169 |
| 14 | Cl | H | Bn | N | 168-169 |
| 15 | Cl | H | Me | CH | 215-216 |
| 16 | Cl | H | Et | CH | 220-221 |
| 17 | Cl | H | Bn | CH | 211-212 |

| | | | | | |
|---|---|---|---|---|---|
| In the table, * indicates an optically active form (R form). | | | | | |

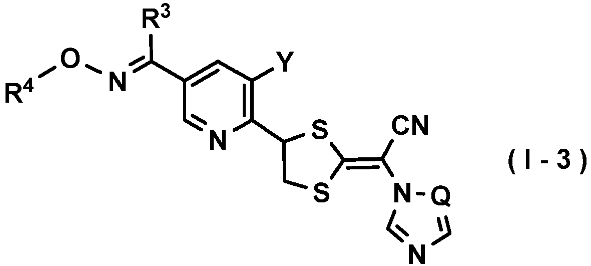

**[Table 3]**

| Compound No. | Y | R³ | R⁴ | Q | Physical property Melting point (°C) |
|---|---|---|---|---|---|
| 18 | F | H | Me | N | NMR |
| 19 | Cl | H | Me | N | 162-163 |
| 20 | F | H | Et | N | 97-98 |
| 21 | Cl | H | Et | N | 150-151 |
| 22 | F | H | Bn | N | 119-120 |
| 23 | Cl | H | Bn | N | 171-172 |
| 24 | Cl | H | Me | CH | 151-152 |
| 25 | Cl | H | Et | CH | 125-126 |
| 26 | Cl | H | Bn | CH | 131-132 |
| 27 | Cl | NH₂ | Bn | CH | NMR |

**[Table 4]**

| Compound No. | ¹H-NMR(CDCl₃) δ value (ppm) |
|---|---|
| 12 | 8.31 (s, 1H), 8.27 (bs, 1H), 8.24 (d, 1H), 8.09 (s, 1H), 6.66 (s, 1H), 5.67 (dd, 1H), 4.15 (dd, 1H), 3.78 (dd, 1H), 1.54 (s, 9H) |
| 18 | 8.49 (s, 1H), 8.33 (s, 1H), 8.09 (s, 1H), 8.07 (d, 1H), 7.79 (dd, 1H), 5.58 (ddd, 1H), 4.18 (dd, 1H), 4.02 (s, 3H), 3.83 (dd, 1H) |
| 27 | 8.74 (d, 1H), 8.03 (d, 1H), 7.61 (s, 1H), 7.44-7.30 (m, 5H), 7.17-7.15 (m, 1H), 7.04-7.02 (m, 1H), 5.68 (dd, 1H), 5.15 (s, 2H), 4.84 (bs, 2H), 4.19 (dd, 1H), 3.82 (dd, 1H) |

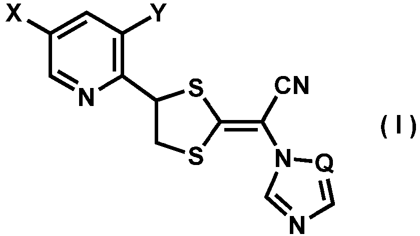

**[Table 5]**

| Compound No. | X | Y | Q | Physical property Melting point (°C) |
|---|---|---|---|---|
| 28 | 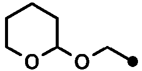 | Cl | CH | NMR |
| 29 | CH₂OH | Cl | CH | 144-145 |
| 30 | CHO | Cl | CH | 93-94 |
| 31 | 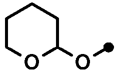 | Cl | CH | 136-137 |
| 32 | 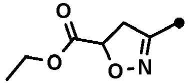 | Cl | CH | 126-127 |
| 33 | MeO | Cl | CH | 168-169 |
| 34 | 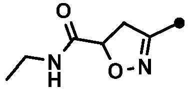 | Cl | CH | 169-170 |
| 35 | 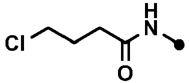 | Cl | CH | 161-162 |

**[Table 6]**

| Compound No. | X | Y | Q | Physical property Melting point (°C) |
|---|---|---|---|---|
| 36 | NH₂HCl | Cl | CH | 186-187 |
| 37 | 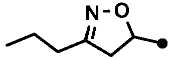 | Cl | CH | 124-126 |
| 38 | NH₂ | Cl | N | 151-152 |
| 39 | CH₂CN | Cl | CH | 103-104 |
| 40 | OH | Cl | CH | 252-253 |
| 41 | OCH₂OCH₃ | Cl | CH | 131-132 |
| 42 | 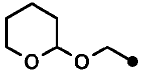 | Cl | N | 103-104 |
| 43 | NH₂ | F | N | 188-189 |
| 44 | CH₂OH | Cl | N | 151-152 |
| 45 | CHO | Cl | N | 147-148 |
| 46 | 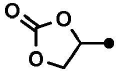 | Cl | CH | NMR |
| 47 | 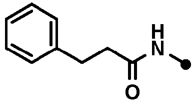 | Cl | CH | NMR |
| 48 | NHCOEt | Cl | CH | NMR |

**[Table 7]**

| Compound No. | X | Y | Q | Physical property Melting point (°C) |
|---|---|---|---|---|
| 49 | NH₂ | Cl | N | 192-193 |
| 50 | 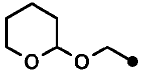 | F | N | NMR |
| 51 | CH₂OH | F | N | 201-202 |
| 52 | CHO | F | N | 126-127 |
| 53* | NH₂ | Cl | N | 221-222 |
| 54** | NH₂ | Cl | N | 230-231 |
| 55 | NH₂ | CN | N | 236-237 |
| 56 | CN | F | N | 189-190 |
| 57 | NH₂ | Me | N | 168-169 |
| 58** | NH₂ | F | N | 215-216 |
| 59* | NH₂ | F | N | 215-216 |
| 60 | CN | Cl | CH | 179-180 |
| 61 | CN | Cl | N | 167-168 |
| 62 | CF₃ | Cl | CH | 132-133 |
| 63 | CF₃ | Cl | N | 148-149 |
| 64 | NHCOMe | Cl | CH | 181-184 |

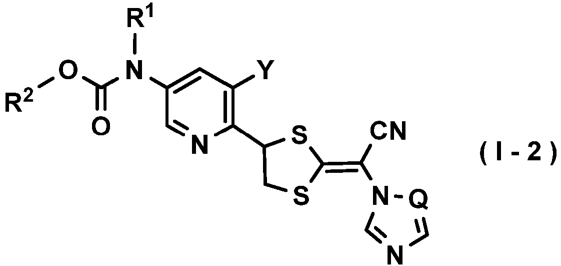

**[Table 8]**

| Compound No. | Y | R¹ | R² | Q | Physical property Melting point (°C) |
|---|---|---|---|---|---|
| 65 | Cl | H | t-Bu | CH | 202-203 |
| 66 | Cl | H | ClCH₂CH₂ | CH | 174-176 |
| 67 | Cl | H | ClCH₂CH₂CH₂ | CH | 167-169 |
| 68 | Cl | H | MeOCH₂CH₂ | CH | 174-175 |
| 69 | Me | H | Et | CH | 210-213 |
| 70 | F | H | t-Bu | N | 149-150 |
| 71 | F | H | Me | N | 193-194 |
| 72 | CN | H | t-Bu | N | NMR |
| 73 | Cl | H | 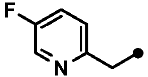 | N | 169-170 |
| 74 | Cl | H | 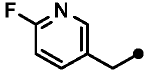 | N | 211-212 |
| 75 | Cl | H | 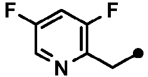 | N | NMR |
| 76** | Cl | H | Me | N | 187-188 |
| 77 | CN | H | Et | N | NMR |
| 78 | CN | H | Bn | N | NMR |
| 79* | Cl | H | Et | N | 174-175 |
| 80** | Cl | H | Et | N | 174-175 |

**[Table 9]**

| Compound No. | Y | R¹ | R² | Q | Physical property Melting point (°C) |
|---|---|---|---|---|---|
| 81 | Me | H | t-Bu | N | NMR |
| 82 | Cl | H | i-Pr | N | 171-172 |
| 83 | Cl | H | i-Bu | N | NMR |
| 84 | Cl | H | n-Pr | N | 134-135 |
| 85 | Cl | H | CF₃CH₂ | N | 182-183 |
| 86 | Cl | H | CHF₂CH₂ | N | 119-120 |
| 87 | Cl | H | c-PrCH₂ | N | 152-153 |
| 88 | Cl | H | c-Pr | N | 98-99 |
| 89 | Cl | H | 4-FBn | N | 184-185 |
| 90 | Me | H | Et | N | 197-198 |
| 91 | Me | H | Bn | N | 153-154 |
| 92* | Cl | H | c-PrCH₂ | N | 156-157 |
| 93** | Cl | H | c-PrCH₂ | N | 156-157 |
| 94* | Cl | H | n-Pr | N | 156-157 |
| 95** | Cl | H | n-Pr | N | 161-162 |
| 96 | Cl | H | 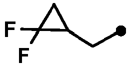 | N | 115-116 |
| 97 | Cl | H | c-BuCH₂ | N | NMR |
| 98 | Cl | H | 3-FBn | N | 189-190 |
| 99 | Cl | H | 2-FBn | N | NMR |

**[Table 10]**

| Compound No. | Y | R¹ | R² | Q | Physical property Melting point (°C) |
|---|---|---|---|---|---|
| 100* | Cl | H | Bn | N | 197-198 |
| 101** | Cl | H | Bn | N | 197-198 |
| 102 | F | H | n-Pr | N | 185-186 |
| 103 | F | H | c-PrCH₂ | N | 203-204 |
| 104** | F | H | Et | N | 177-178 |
| 105** | F | H | Bn | N | 178-179 |
| 106* | F | H | Bn | N | 178-179 |
| 107 | Cl | H | 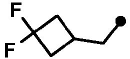 | N | 167-168 |
| 108 | Cl | H | 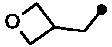 | N | 187-188 |
| 109** | F | H | n-Pr | N | 177-178 |
| 110* | F | H | n-Pr | N | 175-176 |
| 111** | F | H | c-PrCH₂ | N | 205-206 |
| 112* | Cl | H | c-PrCH₂ | N | 205-206 |

**[Table 11]**

| Compound No. | Y | R¹ | R² | Q | Physical property Melting point (°C) |
|---|---|---|---|---|---|
| 113 | Cl | H | 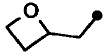 | N | 109-110 |
| 114 | Cl | H | 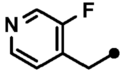 | N | 182-183 |
| 115 | Cl | H | 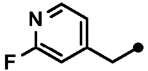 | N | 182-183 |
| 116^{d} | Cl | H | 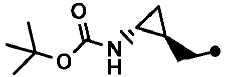 | N | NMR |
| 117 | Cl | H | 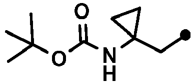 | N | 117-118 |
| 118 | Cl | H | 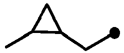 | N | NMR |
| 119 | Cl | H | 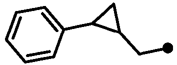 | N | NMR |
| 120 | Cl | H | 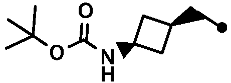 | N | 165-166 |
| 121 | Cl | H | 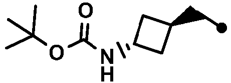 | N | 222-223 |
| 122 | Cl | H | 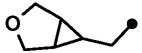 | N | 183-184 |

**[Table 12]**

| Compound No. | Y | R¹ | R² | Q | Physical property Melting point (°C) |
|---|---|---|---|---|---|
| 123 | Cl | H | 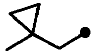 | N | NMR |
| 124 | Cl | H | 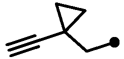 | N | NMR |
| 125 | Cl | H | 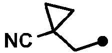 | N | NMR |
| 126 | Cl | H | 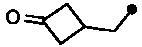 | N | NMR |
| 127 | Cl | H | 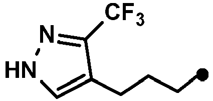 | N | NMR |
| 128 | F | H | 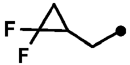 | N | 194-195 |
| 129 | Cl | H | 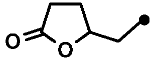 | N | 218-219 |
| 130* | Cl | H | 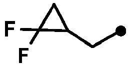 | N | 164-165 |
| 131* | F | H | 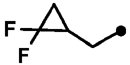 | N | 191-192 |
| 132 | Cl | H | NCCH₂CH₂ | N | 203-204 |

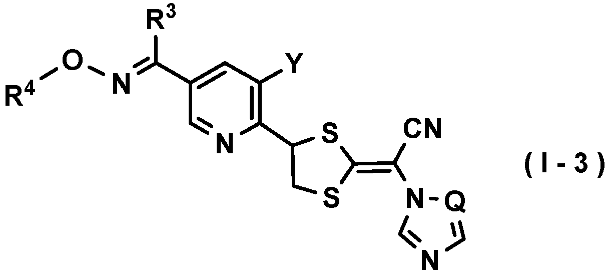

**[Table 13]**

| Compound No. | Y | R³ | R⁴ | Q | Physical property Melting point (°C) |
|---|---|---|---|---|---|
| 133 | Cl | H | H | CH | 244-245 |
| 134 | Cl | NH₂ | Et | CH | 149-150 |
| 135 | Cl | NH₂ | Et | N | 204-205 |
| 136 | Cl | NH₂ | Bn | N | NMR |
| 137 | F | NH₂ | Et | N | 96-97 |
| 138 | F | NH₂ | Bn | N | NMR |
| 139 | Cl | H | c-PrCH₂ | N | 143-144 |
| 140 | F | H | c-PrCH₂ | N | 133-134 |
| 141 | Cl | H | n-Pr | N | 117-118 |
| 142 | Cl | H | i-Pr | N | 161-162 |
| 143 | Cl | H | i-Bu | N | 128-129 |
| 144 | F | H | n-Pr | N | 67-68 |

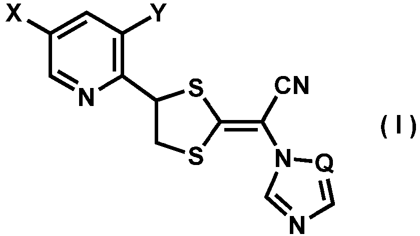

**[Table 141**

| Compound No. | Y | X | Q | Physical property Melting point (°C) |
|---|---|---|---|---|
| 145 | Cl | CH (OH) Me | N | 151-152 |
| 146 | Cl | COMe | N | 150-151 |

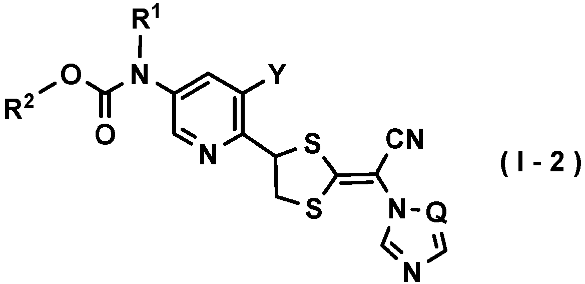

**[Table 151**

| Compound No. | Y | R¹ | R² | Q | Physical property Melting point (°C) |
|---|---|---|---|---|---|
| 147*^{d} | Cl | H | 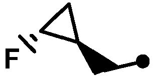 | N | 174-175 |
| 148 | Cl | H | 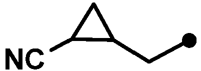 | N | NMR |
| 149*^{d} | F | H | 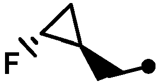 | N | 188-189 |
| 150 | Cl | H | 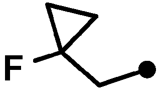 | N | NMR |
| 151 | Cl | H | 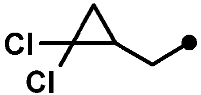 | N | NMR |
| 152* | F | H | 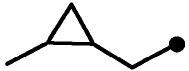 | N | 172-173 |
| 153* | F | H | 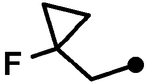 | N | 180-181 |
| 154* | F | H | 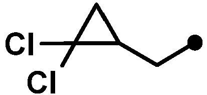 | N | NMR |
| 155* | Cl | H | 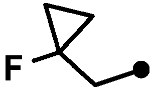 | N | 157-158 |
| 156* | Cl | H | 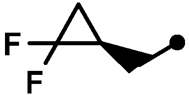 | N | 172-173 |

**[Table 16]**

| Compound No. | Y | R¹ | R² | Q | Physical property Melting point (°C) |
|---|---|---|---|---|---|
| 157* | F | H | 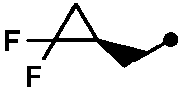 | N | 190-191 |
| 158* | Cl | H | 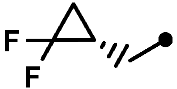 | N | 157-158 |
| 159* | F | H | 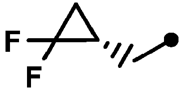 | N | 186-187 |
| 160 ^{d} | Cl | H | 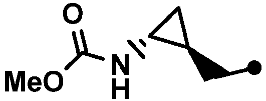 | N | NMR |
| 161* | Cl | H | Me | N | 213-214 |

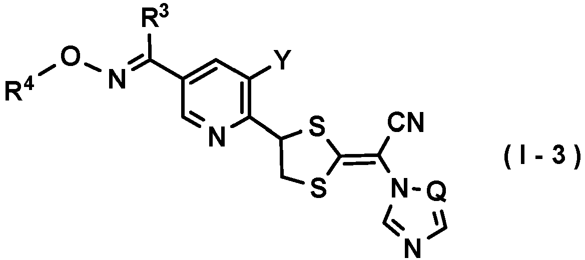

**[Table 17]**

| Compound No. | Y | R³ | R⁴ | Q | Physical property Melting point (°C) |
|---|---|---|---|---|---|
| 162 | Cl | H | 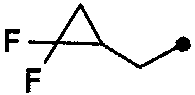 | N | 121-122 |
| 163 | F | H | 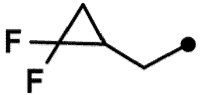 | N | 122-123 |
| 164 | Cl | Me | Et | N | 165-166 |
| 165 | Cl | Me | 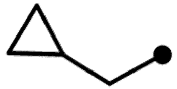 | N | 150-151 |

**[Table 18]**

| Compound No. | ¹H-NMR data (CDCl₃) δ value (ppm) |
|---|---|
| 28 | 8.49 (s, 1H), 7.81-7.79 (m, 1H), 7.62-7.60 (m, 1H), 7.17-7.15 (m, 1H), 7.03 (t, 1H), 5.70 (dd, 1H), 4.83 (d, 1H), 4.74 (t, 1H), 4.54 (d, 1H), 4.19 (dd, 1H), 3.91-3.84 (m, 1H), 3.81 (dd, 1H), 3.61-3.55 (m, 1H), 1.92-1.46 (m, 6H) |
| 46 | 8.53 (d, 1H), 7.84 (dd, 1H), 7.61 (bs, 1H), 7.17 (bs, 1H), 7.03 (bs, 1H), 5.76 (t, 1H), 5.69 (dd, 1H), 4.91 (t, 1H), 4.39 (dd, 1H), 4.20 (dd, 1H), 3.86 (dd, 1H) |
| 47 | 8.39 (d, 1H), 8.23 (d, 1H), 7.60 (s, 1H), 7.36-7.30 (m, 2H), 7.29-7.22 (m, 3H), 7.17 (bs, 1H), 7.16 (s, 1H), 7.03 (s, 1H), 5.67 (dd, 1H), 4.12 (dd, 1H), 3.77 (dd, 1H), 3.07 (t, 2H), 2.74 (t, 2H) |
| 48 | 8.48 (d, 1H), 8.39 (d, 1H), 7.61 (s, 1H), 7.37 (bs, 1H), 7.16 (s, 1H), 7.03 (s, 1H), 5.69 (dd, 1H), 4.14 (dd, 1H), 3.78 (dd, 1H), 2.47 (q, 2H), 1.27 (t, 3H) |
| 50 | 8.40 (bs, 1H), 8.33 (s, 1H), 8.09 (s, 1H), 7.54 (dd, 1H), 5.59 (ddd, 1H), 4.85 (d, 1H), 4.74 (dd, 1H), 4.57 (d, 1H), 4.17 (dd, 1H), 3.87 (ddd, 1H), 3.81 (dd, 1H), 3.61-3.54 (m, 1H), 1.91-1.73 (m, 2H), 1.72-1.50 (m, 4H) |
| 72 | 8.52 (bs, 1H), 8.51 (d, 1H), 8.34 (d, 1H), 8.09 (s, 1H), 6.78 (bs, 1H), 5.68 (dd, 1H), 4.12 (dd, 1H), 3.85 (dd, 1H), 1.54 (s, 9H) |
| 75 | 8.37 (d, 1H), 8.32 (s, 1H), 8.31 (d, 1H), 8.26 (bd, 1H), 8.09 (s, 1H), 7.29 (dd, 1H), 7.01 (bs, 1H), 5.66 (dd, 1H), 5.41 (d, 2H), 4.15 (dd, 1H), 3.80 (dd, 1H) |
| 77 | 8.58 (d, 1H), 8.50 (bs, 1H), 8.34 (s, 1H), 8.10 (s, 1H), 6.88 (bs, 1H), 5.68 (dd, 1H), 4.30 (q, 2H), 4.13 (dd, 1H), 3.86 (dd, 1H), 1.35 (t, 3H) |

**[Table 19]**

| Compound No. | ¹H-NMR data (CDCl₃) δ value (ppm) |
|---|---|
| 78 | 8.57 (d, 1H), 8.51 (bs, 1H), 8.33 (s, 1H), 8.09 (s, 1H), 7.43-7.36 (m, 5H), 6.97 (bs, 1H), 5.68 (dd, 1H), 5.25 (s, 2H), 4.13 (dd, 1H), 3.86 (dd, 1H) |
| 81 | 8.31 (s, 1H), 8.21 (d, 1H), 8.09 (s, 1H), 7.96 (bs, 1H), 6.56 (bs, 1H), 5.45 (dd, 1H), 4.32 (dd, 1H), 3.62 (dd, 1H), 2.44 (s, 3H), 1.53 (s, 9H) |
| 83 | 8.32 (d, 1H), 8.31 (s, 1H), 8.25 (bs, 1H), 8.09 (s, 1H), 6.78 (bs, 1H), 5.67 (dd, 1H), 4.16 (dd, 1H), 4.00 (d, 2H), 3.80 (dd, 1H), 2.00 (sep, 1H), 0.98 (d, 6H) |
| 97 | 8.31 (s, 1H), 8.31 (d, 1H), 8.25 (bs, 1H), 8.09 (s, 1H), 6.77 (bs, 1H), 5.67 (dd, 1H), 4.19 (d, 2H), 4.16 (dd, 1H), 3.79 (dd, 1H), 2.67 (sep, 1H), 2.15-2.05 (m, 2H), 2.03-1.75 (m, 4H) |
| 99 | 8.31 (s, 1H), 8.30 (d, 1H), 8.26 (bs, 1H), 8.08 (s, 1H), 7.44 (ddd, 1H), 7.41-7.34 (m, 1H), 7.17 (ddd, 1H), 7.11 (dd, 1H), 6.82 (bs, 1H), 5.66 (dd, 1H), 5.31 (s, 2H), 4.15 (dd, 1H), 3.79 (dd, 1H) |
| 116 | 8.36-8.33 (m, 1H), 8.32 (s, 1H), 8.25-8.21 (m, 1H), 8.09 (s, 1H), 7.61 (bs, 1H), 5.68 (dd, 1H), 4.76 (bs, 1H), 4.29 (bs, 1H), 4.16 (dd, 1H), 3.84 (bs, 1H), 3.79 (dd, 1H), 2.53 (bs, 1H), 1.44 (s, 9H), 1.40-1.32 (m, 1H), 0.86-0.75 (m, 2H) |
| 118 | 8.33-8.30 (m, 2H), 8.25 (bs, 1H), 8.09 (s, 1H), 6.83 (bs, 1H), 5.67 (dd, 1H), 4.16 (dd, 1H), 4.10-3.98 (m, 2H), 3.80 (dd, 1H), 1.12 (d, 0.6H), 1.08 (d, 2.4H), 1.04-0.70 (m, 2.2H), 0.53-0.46 (m, 0.8H), 0.40-0.34 (m, 0.8H), 0.10-0.05 (m, 0.2H) |

**[Table 20]**

| Compound No. | ¹H-NMR data (CDCl₃) δ value (ppm) |
|---|---|
| 119 | 8.33-8.30 (m, 2H), 8.24 (bs, 1H), 8.09 (s, 1H), 7.30-7.24 (m, 2H), 7.21-7.15 (m, 1H), 7.10-7.06 (m, 2H), 6.83 (bs, 1H), 5.67 (dd, 1H), 4.30-4.23 (m, 1H), 4.19-4.11 (m, 2H), 3.80 (dd, 1H), 1.98-1.92 (m, 1H), 1.60-1.49 (m, 1H), 1.09-1.00 (m, 2H) |
| 123 | 8.32 (d, 1H), 8.32 (s, 1H), 8.25 (bs, 1H), 8.09 (s, 1H), 6.85 (bs, 1H), 5.68 (dd, 1H), 4.16 (dd, 1H), 4.01 (s, 2H), 3.80 (dd, 1H), 1.17 (s, 3H), 0.56-0.52 (m, 2H), 0.45-0.41 (m, 2H) |
| 124 | 8.33 (d, 1H), 8.32 (s, 1H), 8.24 (d, 1H), 8.09 (s, 1H), 6.99 (bs, 1H), 5.67 (dd, 1H), 4.16 (dd, 1H), 4.13 (s, 2H), 3.80 (dd, 1H), 1.97 (s, 1H), 1.12-1.08 (m, 2H), 0.94-0.90 (m, 2H) |
| 125 | 8.37 (d, 1H), 8.32 (s, 1H), 8.21 (bs, 1H), 8.09 (s, 1H), 7.00 (bs, 1H), 5.67 (dd, 1H), 4.20 (dd, 1H), 4.22-4.14 (m, 2H), 3.81 (dd, 1H), 1.44-1.40 (m, 2H), 1.17-1.13 (m, 2H) |
| 126 | 8.35 (d, 1H), 8.32 (s, 1H), 8.23 (bs, 1H), 8.09 (s, 1H), 6.86 (bs, 1H), 5.67 (dd, 1H), 4.39 (d, 2H), 4.16 (dd, 1H), 3.81 (dd, 1H), 3.30-3.20 (m, 2H), 2.99-2.78 (m, 3H) |
| 127 | 10.23 (bs, 1H), 8.34 (d, 1H), 8.32 (s, 1H), 8.23 (bs, 1H), 8.09 (s, 1H), 6.80 (bs, 1H), 5.67 (dd, 1H), 4.26 (t, 2H), 4.17 (dd, 1H), 3.81 (dd, 1H), 2.73 (t, 2H), 2.05-1.96 (m, 2H) |
| 136 | 8.73 (d, 1H), 8.32 (s, 1H), 8.09 (s, 1H), 8.04 (d, 1H), 7.44-7.30 (m, 5H), 5.68 (dd, 1H), 5.15 (s, 2H), 4.84 (bs, 2H), 4.21 (dd, 1H), 3.85 (dd, 1H) |
| 138 | 8.65 (dd, 1H), 8.33 (s, 1H), 8.09 (s, 1H), 7.76 (dd, 1H), 7.44-7.30 (m, 5H), 5.58 (ddd, 1H), 5.15 (s, 2H), 4.84 (bs, 2H), 4.17 (dd, 1H), 3.83 (dd, 1H) |

**[Table 21]**

| Compound No. | ¹H-NMR data (CDCl₃) δ value (ppm) |
|---|---|
| 148 | 8.36-8.34 (m, 1H), 8.32 (s, 1H), 8.21 (bs, 1H), 8.09 (s, 1H), 6.87 (bs, 1H), 5.67 (dd, 1H), 4.32-4.24 (m, 1H), 4.17 (dd, 1H), 3.98-3.90 (m, 1H), 3.81 (dd, 1H), 1.95-1.86 (m, 1H), 1.50-1.36 (m, 2H), 1.13-1.07 (m, 1H) |
| 150 | 8.34 (d, 1H), 8.31 (s, 1H), 8.23 (bs, 1H), 8.09 (s, 1H), 6.91 (bs, 1H), 5.67 (dd, 1H), 4.54-4.41 (m, 2H), 4.17 (dd, 1H), 3.80 (dd, 1H), 1.24-1.14 (m, 2H), 0.91-0.79 (m, 2H) |
| 151 | 8.37-8.34 (m, 1H), 8.32 (s, 1H), 8.24 (bs, 1H), 8.09 (s, 1H), 6.89 (bs, 1H), 5.67 (dd, 1H), 4.57 (dd, 1H), 4.17 (d, 1H), 4.13 (d, 1H), 3.80 (dd, 1H), 2.12-2.02 (m, 1H), 1.76 (dd, 1H), 1.38 (dd, 1H) |
| 154 | 8.32 (s, 1H), 8.24-8.22 (m, 1H), 8.09 (s, 1H), 8.05 (bd, 1H), 6.98 (bs, 1H), 5.56 (ddd, 1H), 4.57 (dd, 1H), 4.13 (dd, 2H), 3.78 (dd, 1H), 2.11-2.02 (m, 1H), 1.76 (dd, 1H), 1.38 (dd, 1H) |
| 160 | 8.35-8.33 (m, 1H), 8.32 (s, 1H), 8.25-8.22 (m, 1H), 8.09 (s, 1H), 7.18 (bs, 1H), 5.67 (dd, 1H), 4.87 (bs, 1H), 4.26 (bs, 1H), 4.16 (dd, 1H), 3.93 (bs, 1H), 3.80 (dd, 1H), 3.66 (s, 3H), 2.62-2.57 (m, 1H), 1.43-1.33 (m, 1H), 0.90-0.79 (m, 2H) |

The compound is an antifungal agent useful for treating fungal infections in humans and animals. Fungal infections are classified into localized fungal infections and systemic fungal infections. Localized fungal infections include tinea pedis and tinea ungums, mainly caused by Trichophyton fungi, known as tinea fungi, seborrheic dermatitis, mainly caused by Malassezia fungi, and vaginal candidiasis, mainly caused by Candida fungi. Systemic fungal infections include candidemia, mainly caused by Candida fungi, and pulmonary aspergillosis, mainly caused by Aspergillus fungi. In addition, the compound can be used to treat localized fungal infections, systemic fungal infections and the like, caused by fungi such as Cryptococcus, Fusarium, Scedosporium, Pneumocystis, Mucor and the like.

Fungi against which the present compound is effective include Trichophyton fungi (Trichophyton spp.) such as Trichophyton rubrum, Trichophyton mentagrophytes, Trichophyton interdigitale and the like, Aspergillus fungi (Aspergillus spp.) such as Aspergillus fumigatus, Aspergillus flavus, Aspergillus niger, Aspergillus nidulans, Aspergillus terreus, Aspergillus clavatus, Aspergillus niveus, Aspergillus versicolor, Aspergillus sydowii, Aspergillus ustus and the like, Malassezia fungi (Malassezia spp.) such as Malassezia restricta, Malassezia globosa and the like, and Candida fungi (Candida spp.) such as Candida albicans, Candida tropicalis, Candida parapsilosis, Candida matapsilosis, Candida orthopsilosis, Candida glabrata, Candida auris, Candida nivariensis, Candida bracarensis, Candida krusei, Candida guilliermondii, Candida guilliermondii var.menbranifaciens, Candida lusitanie, Candida inconspicua, Candida famata, Candida rugosa, Candida dubliniensis, Candida norvegensis, Candida lipolytica, Candida sake, Candida pelliculosa, Candida apicola, Candida zeylanoides, Candida kefyr and the like.

Other fungi include Cryptococcus fungi (Cryptococcus spp.) such as Cryptococcus neoformans, Cryptococcus gatti and the like, Mucor fungi (Mucor spp.) such as Mucor circinelloides, Mucor plumbeus, Mucor racemosus, Mucor ramosissimus, Mucor rouxii and the like, Rhizopus fungi (Rhizopus spp.) such as Rhizopus arrhizus, Rhizopus oryzae, Rhizopus stolonifera, Rhizopus microspores and the like, Rhizomucor fungi (Rhizomucor spp.) such as Rhizomucor pusillus and the like, Lichtheimia fungi (Lichtheimia spp.) such as Lichtheimia corymbifera and the like, Absidia fungi (Absidia spp.) such as Absidia corymbifera, Absidia butleri and the like, Cunnighamella fungi (Cunnighamella spp.) such as Cunnighamella bertholletiae, Cunninghamella echinulate and the like, Conidiobolus fungi (Conidiobolus spp.) such as Conidiobolus brefeldianus, Conidiobolus coronatus, Conidiobolus incongruous, Conidiobolus lamprauges and the like, Trichosporon fungi (Trichosporon spp.) such as Trichosporon asahii, Trichosporon cutaneum, Trichosporon inkin, Trichosporon mucoides and the like, Scedosporium fungi (Scedosporium spp.) such as Scedosporium apiospermum and the like, Fusarium fungi (Fusarium spp.) such as Fusarium oxysporum, Fusarium solani and the like, Pneumocystis fungi (Pneumocystis spp.) such as Pneumocystis jirovecii and the like, and Histoplasma fungi (Histoplasma spp.) such as Histoplasmosis capsulati and the like.

The compounds and pharmacologically acceptable salts thereof of the present invention are used alone or in the form of a composition containing a pharmaceutically acceptable inert carrier or diluent, prepared into a dosage form suitable for oral, intravenous, transdermal, transpulmonary or transvaginal administration, such as powder, granule, tablet, liquid, suspension, emulsion, ointment, cream, gel, lotion, poultice, injection, drip infusion, suppository and the like. After preparation into such a suitable dosage form, a pharmacologically effective amount of the compound and pharmacologically acceptable salts thereof of the present invention can be administered orally, intravenously, transdermally, transpulmonary or transvaginally to treat the above-mentioned localized fungal infections or systemic fungal infections. The dosage varies depending on age, body weight, and mode of administration. For oral, intravenous, transpulmonary or transvaginal administration, the dosage for an adult is usually 0.05 mg or more, preferably 0.5 to 5.0 mg, per kg of body weight per day, administered once or in several divided doses. For transdermal administration, for example, in the case of preparations for application such as ointment, cream, lotion, poultice and the like, the concentration of the active ingredient is 0.001% or more, preferably in the range of 0.1 to 10%, and applied to the skin or nails in the range 30 mg to 100 mg per cm². The compound of the present invention can also be applied in mixture with other antifungal and antibacterial agents, such as Amphotericin B, Trichomycin, Valitoin, Clotrimazole and the like.

### [Example]

Next, production examples, formulation examples and experimental examples are given as specific examples of the present invention, but the present invention is not limited to these. The parts indicate parts by weight.

### Production Example 1 Production of methyl (E)-(5-chloro-6-{2-[cyano(1H-imidazol-1-yl)methylene]-1,3-dithiolan-4-yl}pyridin-3-yl)carbamate (Compound No.15)

### 1-1) Production of tertiary-butyl (5-chloro-6-vinylpyridin-3-yl)carbamate

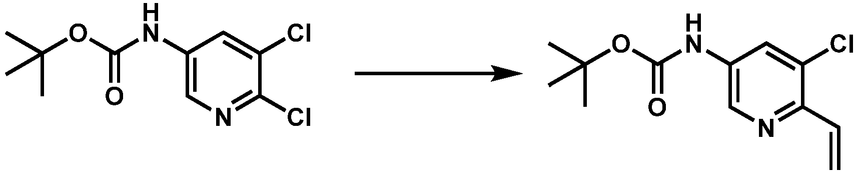

Tertiary-butyl (5,6-dichloropyridin-3-yl)carbamate (6.1 g, 23 mmol) was dissolved in 1,2-dimethoxyethane (50 mL) and water (25 mL), and then sodium carbonate (9.9 g, 93 mmol), [1,1'-bis(diphenylphosphino)ferrocene]palladium(II) dichloride acetone adduct (0.91 g, 1.2 mmol) and potassium vinyltrifluoroborate (5.6 g, 42 mmol) were added thereto, and the mixture was stirred with heating under reflux for 3.5 hr. Saturated aqueous sodium chloride solution was added thereto, and the mixture was extracted threetimes with ethyl acetate. The organic layer was dried over anhydrous sodium sulfate, and concentrated under reduced pressure. The obtained residue was purified by silica gel column chromatography to give tertiary-butyl (5-chloro-6-vinylpyridin-3-yl)carbamate (5.8 g).
yield: 98%
¹H-NMR(CDCl₃) δ value (ppm) :
   8.21(d,1H),8.16(bs,1H),7.17(dd,1H),6.56(bs,1H),6.33(dd,1H),5.49(dd , 1H) 1. 53 (s, 9H)

### 1-2) Production of tertiary-butyl [5-chloro-6-(1,2-dihydroxyethyl)pyridin-3-yl]carbamate

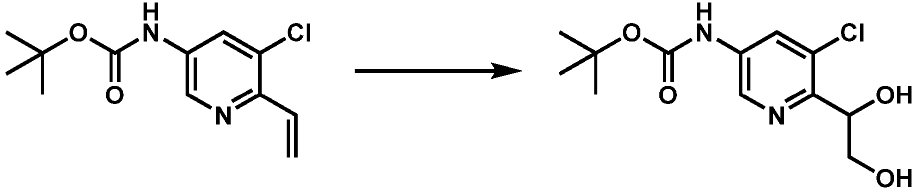

Tertiary-butyl (5-chloro-6-vinylpyridin-3-yl)carbamate (5.8 g, 23 mmol) was dissolved in acetone (45 mL) and water (4.5 mL), and then 4-methylmorpholine N-oxide (50% aqueous solution, 5.8 g, 25 mmol) and osmium tetroxide (0.1M aqueous solution, 1.1 mL, 0.11 mmol) were added thereto, and the mixture was stirred at room temperature for 26 hr. Sodium dithionite (2.0 g, 12 mmol) and ethyl acetate were added thereto, and then the mixture was filtered through silica gel and Celite, and the filtrate was concentrated under reduced pressure. The obtained residue was purified by silica gel column chromatography to give tertiary-butyl [5-chloro-6-(1,2-dihydroxyethyl)pyridin-3-yl]carbamate (5.2 g).
yield: 80%
¹H-NMR(CDCl₃) δ value (ppm) :
   8.28(bs,1H),8.14(bs,1H),6.56(bs,1H),5.07(bs,1H),4.28(bs,1H),3.96(d d,1H),3.66(dd,1H),2.44(bs,1H),1.53(s,9H)

### 1-3) Production of tertiary-butyl (E)-(5-chloro-6-{2-[cyano(1H-imidazol-1-yl)methylene]-1,3-dithiolan-4-yl}pyridin-3-yl)carbamate

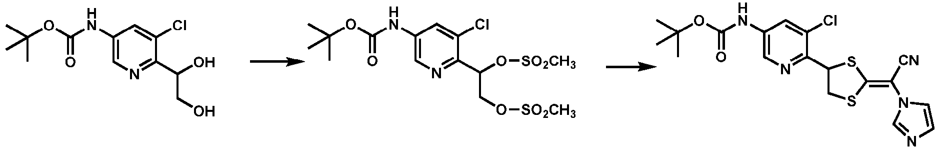

Tertiary-butyl [5-chloro-6-(1,2-dihydroxyethyl)pyridin-3-yl]carbamate (0.50 g, 1.7 mmol) and triethylamine (0.42 g, 4.2 mmol) were dissolved in chloroform (5 mL), and then methanesulfonyl chloride (0.44 g, 3.8 mmol) was added thereto under ice-cooling, and the mixture was stirred at room temperature for 3 hr. The reaction solution was concentrated under reduced pressure, the obtained residue was suspended in ethyl acetate, and the suspension was filtered through silica gel and Celite. The obtained filtrate was concentrated under reduced pressure to give 1-{5-[(tertiary-butoxycarbonyl)amino]-3-chloropyridin-2-yl}ethane-1,2-diyl dimethanesulfonate as a crude product.

Potassium hydroxide (powder, 0.29 g, 5.2 mmol) was suspended in dimethyl sulfoxide (4 mL), and then 2-(1H-imidazol-1-yl)acetonitrile (0.28 g, 2.6 mmol) and a dimethyl sulfoxide solution (4 mL) of carbon disulfide (0.20 g, 2.6 mmol) were added thereto, and the mixture was stirred at room temperature for 3 hr. The obtained reaction solution was added to a dimethyl sulfoxide solution (8 mL) of 1-{5-[(tertiary-butoxycarbonyl)amino]-3-chloropyridin-2-yl}ethane-1,2-diyl dimethanesulfonate, and the mixture was stirred at room temperature for 16 hr. Water was added thereto, and the mixture was extracted threetimes with ethyl acetate. The organic layer was dried over anhydrous sodium sulfate, and concentrated under reduced pressure. The obtained residue was purified by silica gel column chromatography to give tertiary-butyl (E)-(5-chloro-6-{2-[cyano(1H-imidazol-1-yl)methylene]-1,3-dithiolan-4-yl}pyridin-3-yl)carbamate (0.29 g).
yield: 39%
melting point: 202-203°C

### 1-4) Production of (E)-2-[4-(5-amino-3-chloropyridin-2-yl)-1,3-dithiolan-2-ylidene]-2-(1H-imidazol-1-yl)acetonitrile hydrochloride

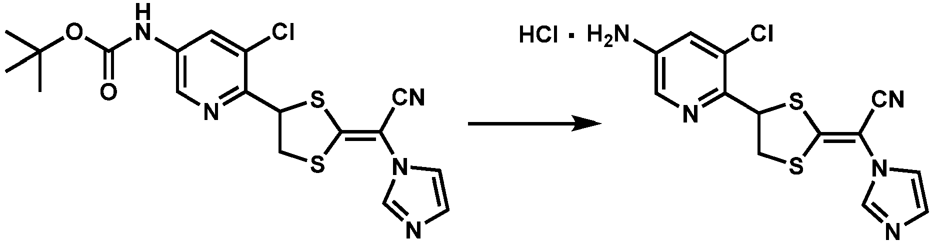

Tertiary-butyl (E)-(5-chloro-6-{2-[cyano(1H-imidazol-1-yl)methylene]-1,3-dithiolan-4-yl}pyridin-3-yl)carbamate (50 g, 0.11mol) was dissolved in methanol (500 mL), and then hydrogen chloride methanol solution (250 mL) was added thereto, and the mixture was stirred at 25°C for 12 hr. The resulting solid was filtered, and the filtered solid was dried under reduced pressure to give (E)-2-[4-(5-amino-3-chloropyridin-2-yl)-1,3-dithiolan-2-ylidene]-2-(1H-imidazol-1-yl)acetonitrile hydrochloride (42 g).
yield: 98%
melting point: 186-187°C

### 1-5) Production of methyl (E)-(5-chloro-6-{2-[cyano(1H-imidazol-1-yl)methylene]-1,3-dithiolan-4-yl}pyridin-3-yl)carbamate

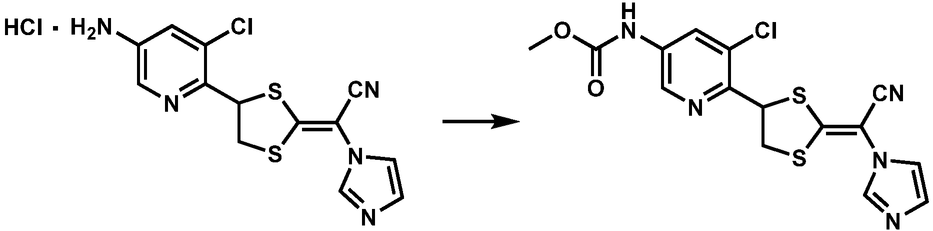

(E)-2-[4-(5-Amino-3-chloropyridin-2-yl)-1,3-dithiolan-2-ylidene]-2-(1H-imidazol-1-yl)acetonitrile hydrochloride (0.10 g, 0.27 mmol) was dissolved in pyridine (2 mL), and then chloromethyl formate (51 mg, 0.54 mmol) was added thereto under ice-cooling, and the mixture was stirred at room temperature for 51 hr. Saturated aqueous sodium hydrogencarbonate solution was added thereto, and the mixture was extracted threetimes with ethyl acetate. The organic layer was dried over anhydrous sodium sulfate, and concentrated under reduced pressure. The obtained residue was suspended in ethyl acetate, and the solid was filtered. The filtered solid was air-dried to give methyl (E)-(5-chloro-6-{2-[cyano(1H-imidazol-1-yl)methylene]-1,3-dithiolan-4-yl}pyridin-3-yl)carbamate (71 mg).
yield: 67%
melting point: 215-216°C

### Production Example 2 Production of (E)-2-(4-{3-chloro-5-[(E)-(ethoxyimino)methyl]pyridin-2-yl}-1,3-dithiolan-2-ylidene)-2-(1H-imidazol-1-yl)acetonitrile (Compound No.25)

### 2-1) Production of 3-chloro-5-{[(tetrahydro-2H-pyran-2-yl)oxy]methyl}-2-vinylpyridine

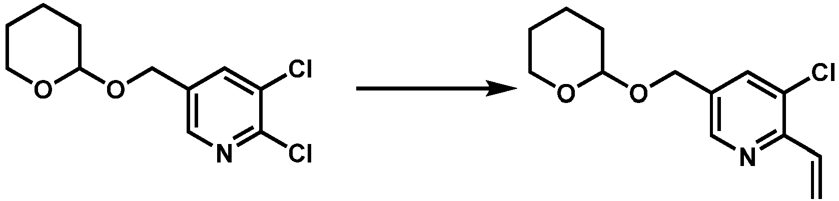

2,3-Dichloro-5-{[(tetrahydro-2H-pyran-2-yl)oxy]methyl}pyridine (8.0 g, 31 mmol) was dissolved in 1,2-dimethoxyethane (60 mL) and water (30 mL), and then sodium carbonate (16 g, 0.15 mol), tetrakis(triphenylphosphine)palladium(0) (2.8 g, 2.4 mmol) and potassium vinyltrifluoroborate (10 g, 77 mmol) were added thereto, and the mixture was stirred with heating under reflux for 6.5 hr. Saturated aqueous sodium chloride solution was added thereto, and the mixture was extracted threetimes with ethyl acetate. The organic layer was dried over anhydrous sodium sulfate, and concentrated under reduced pressure. The obtained residue was purified by silica gel column chromatography to give 3-chloro-5-{[(tetrahydro-2H-pyran-2-yl)oxy]methyl}-2-vinylpyridine (6.6 g).
yield: 86%
¹H-NMR(CDCl₃) δ value (ppm) :
   8.45(d,1H),7.69(d,1H),7.23(dd,1H),6.46(dd,1H),5.58(dd,1H),4.77(d,1 H),4.71(t,1H),4.50(d,1H),3.92-3.84(m,1H),3.60-3.52(m,1H),1.91-1.49(m,6H)

### 2-2) Production of 1-(3-chloro-5-{[(tetrahydro-2H-pyran-2-yl)oxy]methyl}pyridin-2-yl)ethane-1,2-diol

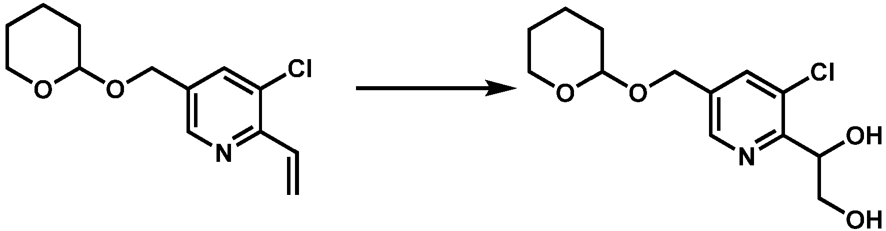

3-Chloro-5-{[(tetrahydro-2H-pyran-2-yl)oxy]methyl}-2-vinylpyridine (6.6 g, 26 mmol) was dissolved in acetone (55 mL) and water (13 mL), and then 4-methylmorpholine N-oxide (50% aqueous solution, 9.2 g, 39 mmol) and osmium tetroxide (0.1M aqueous solution, 5.2 mL, 0.52 mmol) were added thereto, and the mixture was stirred at room temperature for 52 hr. Sodium dithionite (4.6 g, 26 mmol) and ethyl acetate were added thereto, the mixture was filtered through silica gel and Celite, and the obtained filtrate was concentrated under reduced pressure. The obtained residue was purified by silica gel column chromatography to give 1-(3-chloro-5-{[(tetrahydro-2H-pyran-2-yl)oxy]methyl}pyridin-2-yl)ethane-1,2-diol (6.0 g).
yield: 80%
¹H-NMR(CDCl₃) δ value (ppm) :
   8.45(d,1H),7.75(d,1H),5.14-5.08(m,1H),4.80(d,1H),4.72(t,1H),4.52(d,1H),4.52(d,1H),4.04-3.95(m,1H),3.91-3.83(m,1H),3.73-3.65(m,1H),3.61-3.53(m,1H),2.45(bs,1H),1.92-1.49(m,6H)

### 2-3) Production of (E)-2-[4-(3-chloro-5-{[(tetrahydro-2H-pyran-2-yl)oxy]methyl}pyridin-2-yl)-1,3-dithiolan-2-ylidene]-2-(1H-imidazol-1-yl)acetonitrile

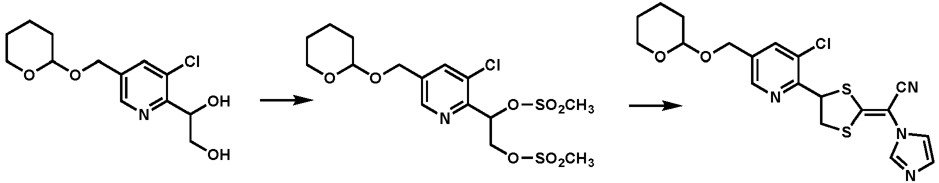

1-(3-Chloro-5-{[(tetrahydro-2H-pyran-2-yl)oxy]methyl}pyridin-2-yl)ethane-1,2-diol (2.0 g, 7.0 mmol) and triethylamine (1.7 g, 17 mmol) were dissolved in chloroform (15 mL), and then methanesulfonyl chloride (1.8 g, 15 mmol) was added thereto under ice-cooling, and the mixture was stirred at room temperature for 23 hr. The reaction solution was concentrated under reduced pressure, the obtained residue was suspended in ethyl acetate, and the suspension was filtered through silica gel and Celite. The obtained filtrate was concentrated under reduced pressure to give 1-(3-chloro-5-{[(tetrahydro-2H-pyran-2-yl)oxy]methyl}pyridin-2-yl)ethane-1,2-diyl dimethanesulfonate as a crude product. Potassium hydroxide (powder, 1.2 g, 21 mmol) was suspended in dimethyl sulfoxide (7 mL), and then 2-(1H-imidazol-1-yl)acetonitrile (1.1 g, 10 mmol) and a dimethyl sulfoxide solution (7 mL) of carbon disulfide (0.79 g, 10 mmol) were added thereto, and the mixture was stirred at room temperature for 3.5 hr. The obtained reaction solution was added to a dimethyl sulfoxide solution (14 mL) of 1-(3-chloro-5-{[(tetrahydro-2H-pyran-2-yl)oxy]methyl}pyridin-2-yl)ethane-1,2-diyl dimethanesulfonate, and the mixture was stirred at room temperature for 27 hr. Water was added thereto, and the mixture was extracted threetimes with ethyl acetate. The organic layer was dried over anhydrous sodium sulfate, and concentrated under reduced pressure. The obtained residue was purified by silica gel column chromatography to give (E)-2-[4-(3-chloro-5-{[(tetrahydro-2H-pyran-2-yl)oxy]methyl}pyridin-2-yl)-1,3-dithiolan-2-ylidene]-2-(1H-imidazol-1-yl)acetonitrile (0.60 g).
yield: 20%
¹H-NMR(CDCl₃) δ value (ppm) :
   8.49(s,1H),7.81-7.79(m,1H),7.62-7.60(m,1H),7.17-7.15(m,1H),7.03(t,1H),5.70(dd,1H),4.83(d,1H),4.74(t,1H),4.54(d,1H) ,4.19(dd,1H),3.91-3.84(m,1H),3.81(dd,1H),3.61-3.55(m,1H),1.92-1.46(m,6H)

### 2-4) Production of (E)-2-{4-[3-chloro-5-(hydroxymethyl)pyridin-2-yl]-1,3-dithiolan-2-ylidene}-2-(1H-imidazol-1-yl)acetonitrile

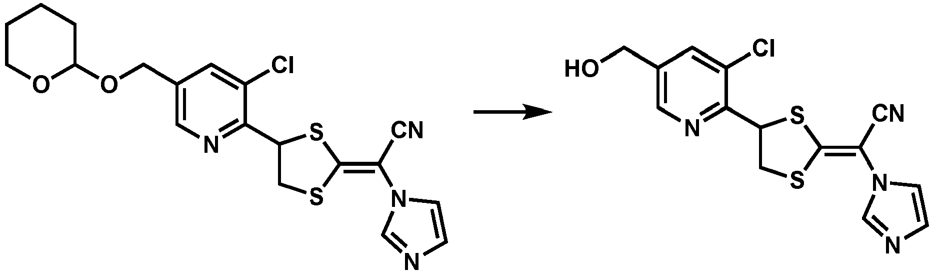

(E)-2-[4-(3-Chloro-5-{[(tetrahydro-2H-pyran-2-yl)oxy]methyl}pyridin-2-yl)-1,3-dithiolan-2-ylidene]-2-(1H-imidazol-1-yl)acetonitrile (0.56 g, 1.3 mmol) was dissolved in methanol (5 mL), and then p-toluenesulfonic acid monohydrate (0.49 g, 2.6 mmol) was added thereto, and the mixture was stirred at room temperature for 33 hr. Saturated aqueous sodium hydrogencarbonate solution and saturated aqueous sodium chloride solution were added thereto, and the mixture was extracted threetimes with ethyl acetate. The organic layer was dried over anhydrous sodium sulfate, and concentrated under reduced pressure. The obtained residue was purified by silica gel column chromatography to give (E)-2-{4-[3-chloro-5-(hydroxymethyl)pyridin-2-yl]-1,3-dithiolan-2-ylidene}-2-(1H-imidazol-1-yl)acetonitrile (0.32 g).
yield: 72%
melting point: 144-145°C

### 2-5) Production of (E)-2-{4-[3-chloro-5-(oxomethyl)pyridin-2-yl]-1,3-dithiolan-2-ylidene}-2-(1H-imidazol-1-yl)acetonitrile

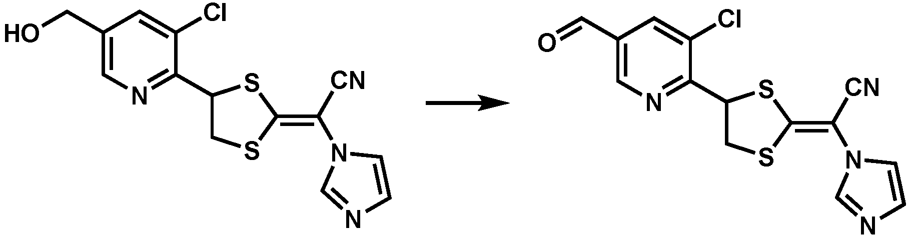

Oxalyl chloride (0.27 g, 2.1 mmol) was dissolved in dichloromethane (4 mL), and then a dichloromethane solution (1 mL) of dimethyl sulfoxide (0.33 g, 4.3 mmol) was added thereto at - 70°C, and the mixture was stirred at the same temperature for 20 min. A solution of (E)-2-{4-[3-chloro-5-(hydroxymethyl)pyridin-2-yl]-1,3-dithiolan-2-ylidene}-2-(1H-imidazol-1-yl)acetonitrile (0.50 g, 1.4 mmol) in dimethyl sulfoxide (2 mL)-dichloromethane (2 mL) was added thereto at -70°C, and the mixture was stirred at the same temperature for 1.5 hr. A dichloromethane solution (1 mL) of triethylamine (0.65 g, 6.4 mmol) was added thereto at -70°C, and the mixture was stirred at the same temperature for 20 min, followed by at room temperature for 2 hr. Water was added thereto, and the mixture was extracted threetimes with ethyl acetate. The organic layer was dried over anhydrous sodium sulfate, and concentrated under reduced pressure. The obtained residue was purified by silica gel column chromatography to give (E)-2-{4-[3-chloro-5-(oxomethyl)pyridin-2-yl]-1,3-dithiolan-2-ylidene}-2-(1H-imidazol-1-yl)acetonitrile (0.46 g).
yield: 93%
melting point: 93-94°C

2-6) Production of (E)-2-(4-{3-chloro-5-[(E)-(ethoxyimino)methyl]pyridin-2-yl}-1,3-dithiolan-2-ylidene)-2-(1H-imidazol-1-yl)acetonitrile

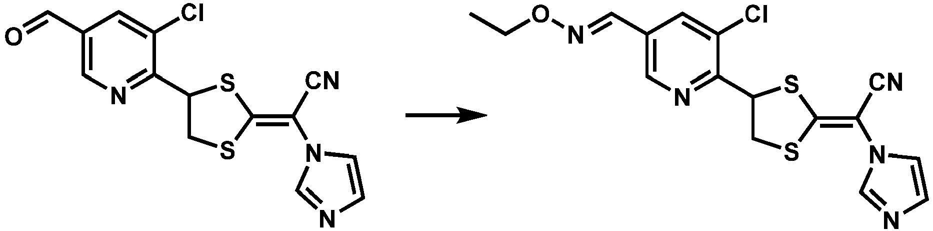

(E)-2-{4-[3-Chloro-5-(oxomethyl)pyridin-2-yl]-1,3-dithiolan-2-ylidene}-2-(1H-imidazol-1-yl)acetonitrile (0.10 g, 0.29 mmol) was dissolved in methanol (3 mL) and tetrahydrofuran (3 mL), and then O-ethylhydroxylamine hydrochloride (31 mg, 0.32 mmol) was added thereto, and the mixture was stirred at room temperature for 27 hr. Saturated aqueous sodium hydrogencarbonate solution was added thereto, and the mixture was extracted threetimes with ethyl acetate. The organic layer was dried over anhydrous sodium sulfate, and concentrated under reduced pressure. The obtained residue was suspended in methyl tertiary-butyl ether, and the solid was filtered. The filtered solid was air-dried to give (E)-2-(4-{3-chloro-5-[(E)-(ethoxyimino)methyl]pyridin-2-yl}-1,3-dithiolan-2-ylidene)-2-(1H-imidazol-1-yl)acetonitrile (78 mg).
yield: 69%
melting point: 125-126°C

### Production Example 3 Production of (E)-2-{4-[3-chloro-5-(5-propyl-4,5-dihydroisoxazol-3-yl)pyridin-2-yl]-1,3-dithiolan-2-ylidene}-2-(1H-imidazol-1-yl)acetonitrile (Compound No.6)

### 3-1) Production of 3-(5,6-dichloropyridin-3-yl)-5-propyl-4,5-dihydroisoxazole

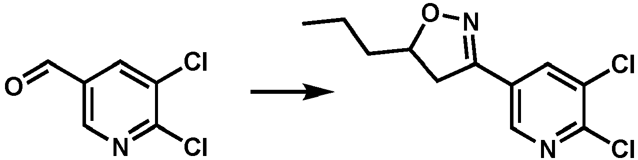

5,6-Dichloronicotinaldehyde (0.95 g, 5.4 mmol) was dissolved in N,N-dimethylformamide (12 mL), and then hydroxylamine hydrochloride (0.39 g, 5.6 mmol) and triethylamine (0.57 g, 5.6 mmol) were added thereto, and the mixture was stirred at room temperature for 1.5 hr. N-Chlorosuccinimide (0.79 g, 5.9 mmol) was added thereto, and the mixture was stirred at room temperature for 2.5 hr. A N,N-dimethylformamide solution (2 mL) of 1-pentene (0.45 g, 6.4 mmol) and triethylamine (0.65 g, 6.4 mmol) was added thereto, and the mixture was stirred at room temperature for 15 hr. Water was added thereto, and the mixture was extracted threetimes with ethyl acetate. The organic layer was dried over anhydrous sodium sulfate, and concentrated under reduced pressure. The obtained residue was purified by silica gel column chromatography to give 3-(5,6-dichloropyridin-3-yl)-5-propyl-4,5-dihydroisoxazole (0.30 g).
yield: 21%
¹H-NMR(CDCl₃) δ value (ppm) :
   8.46(d,1H),8.15(d,1H),4.87-4.78(m,1H),3.37(dd,1H),2.94(dd,1H),1.85-1.75(m,1H),1.68-1.39(m,3H),0.99(t,3H)

### 3-2) Production of 3-(5-chloro-6-vinylpyridin-3-yl)-5-propyl-4,5-dihydroisoxazole

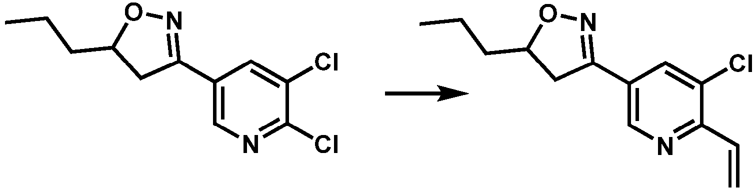

3-(5,6-Dichloropyridin-3-yl)-5-propyl-4,5-dihydroisoxazole (0.29 g, 1.1 mmol) was dissolved in 1,2-dimethoxyethane (4 mL) and water (2 mL), and then sodium carbonate (0.35 g, 3.3 mmol), tetrakis(triphenylphosphine)palladium(0) (64 mg, 55 pmol) and potassium vinyltrifluoroborate (0.22 g, 1.7 mmol) were added thereto, and the mixture was stirred with heating under reflux for 50 min. Saturated aqueous sodium chloride solution was added thereto, and the mixture was extracted threetimes with ethyl acetate. The organic layer was dried over anhydrous sodium sulfate, and concentrated under reduced pressure. The obtained residue was purified by silica gel column chromatography to give 3-(5-chloro-6-vinylpyridin-3-yl)-5-propyl-4,5-dihydroisoxazole (0.23 g).
yield: 83%
¹H-NMR(CDCl₃) δ value (ppm) :
   8.69(d,1H),7.98(d,1H),7.25(dd,1H),6.54(dd,1H),5.65(dd,1H),4.85-4.76(m,1H),3.38(dd,1H),2.94(dd,1H),1.85-1.75(m,1H),1.68-1.37(m,3H),0.99(t,3H)

### 3-3) Production of 1-[3-chloro-5-(5-propyl-4,5-dihydroisoxazol-3-yl)pyridin-2-yl]ethane-1,2-diol

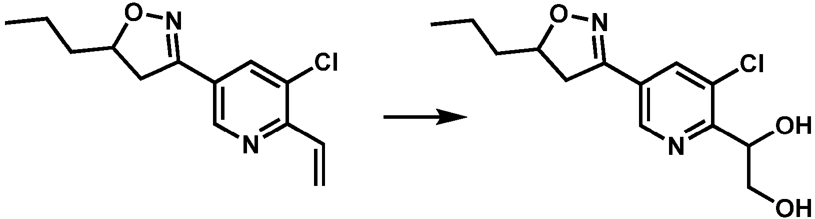

3-(5-Chloro-6-vinylpyridin-3-yl)-5-propyl-4,5-dihydroisoxazole (0.23 g, 0.91 mmol) was dissolved in acetone (10 mL) and water (1 mL), and then 4-methylmorpholine N-oxide (50% aqueous solution, 0.24 g, 1.0 mmol) and osmium tetroxide (0.1M aqueous solution, 91 µL, 9.1pmol) were added thereto, and the mixture was stirred at room temperature for 28 hr. Sodium dithionite (80 mg, 0.46 mmol) and ethyl acetate were added thereto, and then the mixture was filtered through silica gel and Celite, and the obtained filtrate was concentrated under reduced pressure. The obtained residue was purified by silica gel column chromatography to give 1-[3-chloro-5-(5-propyl-4,5-dihydroisoxazol-3-yl)pyridin-2-yl]ethane-1,2-diol (0.21 g).
yield: 79%
¹H-NMR(CDCl₃) δ value (ppm) :
   8.67(dd,1H),8.06(dd,1H),5.17-5.12(m,1H),4.87-4.78(m,1H),4.44(d,1H),4.05-3.97(m,1H),3.77-3.69(m,1H),3.38(ddd,1H),2.95(ddd,1H),2.38(bs,1H),1.86-1.74(m,1H),1.69-1.38(m,3H),0.99(t,3H)

### 3-4) Production of (E)-2-{4-[3-chloro-5-(5-propyl-4,5-dihydroisoxazol-3-yl)pyridin-2-yl]-1,3-dithiolan-2-ylidene}-2-(1H-imidazol-1-yl)acetonitrile

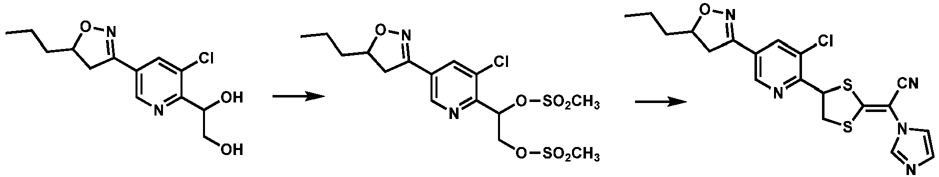

1-[3-Chloro-5-(5-propyl-4,5-dihydroisoxazol-3-yl)pyridin-2-yl]ethane-1,2-diol (0.19 g, 0.67 mmol) and triethylamine (0.16 g, 1.6 mmol) were dissolved in chloroform (5 mL), and then methanesulfonyl chloride (0.17 g, 1.5 mmol) was added thereto under ice-cooling, and the mixture was stirred at room temperature for 2.5 hr. The reaction solution was concentrated under reduced pressure, the obtained residue was suspended in ethyl acetate, and the suspension was filtered through silica gel and Celite. The obtained filtrate was concentrated under reduced pressure to give 1-[3-chloro-5-(5-propyl-4,5-dihydroisoxazol-3-yl)pyridin-2-yl]ethane-1,2-diyl dimethanesulfonate as a crude product. Potassium hydroxide (powder, 0.11 g, 2.0 mmol) was suspended in dimethyl sulfoxide (2 mL), and then 2-(1H-imidazol-1-yl)acetonitrile (0.11 g, 1.0 mmol) and a dimethyl sulfoxide solution (2 mL) of carbon disulfide (76 mg, 1.0 mmol) were added thereto, and the mixture was stirred at room temperature for 2 hr. The obtained reaction solution was added to a dimethyl sulfoxide solution (4 mL) of 1-[3-chloro-5-(5-propyl-4,5-dihydroisoxazol-3-yl)pyridin-2-yl]ethane-1,2-diyl dimethanesulfonate, and the mixture was stirred at room temperature for 19 hr. Water was added thereto, and the mixture was extracted threetimes with ethyl acetate. The organic layer was dried over anhydrous sodium sulfate, and concentrated under reduced pressure. The obtained residue was purified by silica gel column chromatography to give (E)-2-{4-[3-chloro-5-(5-propyl-4,5-dihydroisoxazol-3-yl)pyridin-2-yl]-1,3-dithiolan-2-ylidene}-2-(1H-imidazol-1-yl)acetonitrile (0.17 g).
yield: 58%
melting point: 133-134°C

### Production Example 4 Production of (Z)-N'-(benzyloxy)-5-chloro-6-{(E)-2-[cyano(1H-imidazol-1-yl)methylene]-1,3-dithiolan-4-yl}nicotinimidamide (Compound No.27)

### 4-1) Production of 5-chloro-6-vinylnicotinonitrile

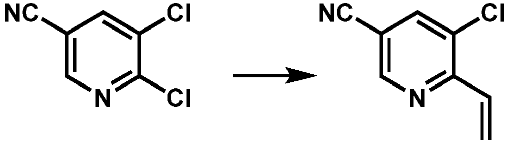

5,6-Dichloronicotinonitrile (0.62 g, 3.6 mmol) was dissolved in 1,2-dimethoxyethane (8 mL) and water (4 mL), and then sodium carbonate (1.5 g, 14 mmol), tetrakis(triphenylphosphine)palladium(0) (0.21 g, 0.18 mmol) and potassium vinyltrifluoroborate (0.96 g, 7.2 mmol) were added thereto, and the mixture was stirred with heating under reflux for 2.5 hr. Saturated aqueous sodium chloride solution was added thereto, and the mixture was extracted threetimes with ethyl acetate. The organic layer was dried over anhydrous sodium sulfate, and concentrated under reduced pressure. The obtained residue was purified by silica gel column chromatography to give 5-chloro-6-vinylnicotinonitrile (0.45 g).
yield: 76%
¹H-NMR(CDCl₃) δ value (ppm) :
   8.71(d,1H),7.93(d,1H),7.25(dd,1H),6.67(dd,1H),5.81(dd,1H)

### 4-2) Production of 5-chloro-6-(1,2-dihydroxyethyl)nicotinonitrile

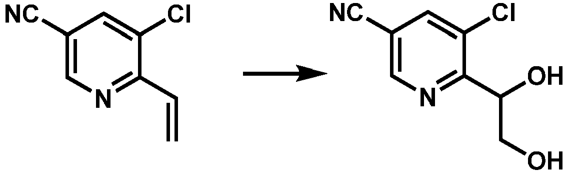

5-Chloro-6-vinylnicotinonitrile (2.2 g, 14 mmol) was dissolved in acetone (30 mL) and water (6 mL), and then 4-methylmorpholine N-oxide (50% aqueous solution, 3.5 g, 15 mmol) and osmium tetroxide (0.1M aqueous solution, 0.68 mL, 68 pmol) were added thereto, and the mixture was stirred at room temperature for 24 hr. Sodium dithionite (1.2 g, 6.8 mmol) and ethyl acetate were added thereto, and then the mixture was filtered through silica gel and Celite, and the obtained filtrate was concentrated under reduced pressure. The obtained residue was purified by silica gel column chromatography to give 5-chloro-6-(1,2-dihydroxyethyl)nicotinonitrile (2.2 g).
yield: 83%
¹H-NMR(CDCl₃) δ value (ppm) :
   8.77(d,1H),8.00(d,1H),5.19(ddd,1H),4.24(d,1H),4.02(ddd,1H),3.78(dd d,1H),2.27(dd,1H)

### 4-3) Production of (Z)-N'-(benzyloxy)-5-chloro-6-(1,2-dihydroxyethyl)nicotinimidamide

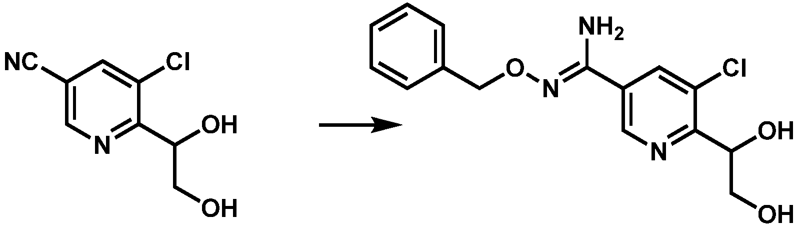

5-Chloro-6-(1,2-dihydroxyethyl)nicotinonitrile (0.50 g, 2.5 mmol) was dissolved in ethanol (10 mL) and water (2.5 mL), and then O-benzyl hydroxylamine hydrochloride (0.80 g, 5.0 mmol), triethylamine (0.89 g, 8.8 mmol) and thioglycol acid (0.23 g, 2.5 mmol) were added thereto, and the mixture was stirred with heating under reflux for 11 hr. Ethyl acetate was added thereto, and then the mixture was filtered through silica gel and Celite, and the obtained filtrate was concentrated under reduced pressure. The obtained residue was suspended in ethyl acetate-hexane (2:1), and the solid was filtered. The filtered solid was air-dried to give (Z)-N'-(benzyloxy)-5-chloro-6-(1,2-dihydroxyethyl)nicotinimidamide (0.54 g).
yield: 66%
¹H-NMR(CDCl₃) δ value (ppm) :
   8.71(d,1H),7.99(d,1H),7.44-7.30(m,5H),5.15(s,2H),5.13(ddd,1H),4.83(bs,2H),4.44(d,1H),3.99(ddd ,1H),3.69(ddd,1H),2.39(dd,1H)

### 4-4) Production of (Z)-N'-(benzyloxy)-5-chloro-6-{(E)-2-[cyano(1H-imidazol-1-yl)methylene]-1,3-dithiolan-4-yl}nicotinimidamide

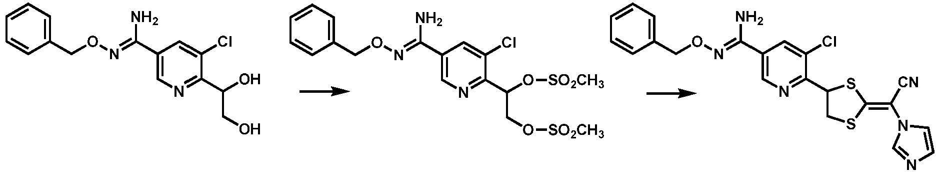

(Z)-N'-(Benzyloxy)-5-chloro-6-(1,2-dihydroxyethyl)nicotinimidamide (0.20 g, 0.63 mmol) and triethylamine (0.15 g, 1.5 mmol) were dissolved in chloroform (5 mL), and then methanesulfonyl chloride (0.16 g, 1.4 mmol) was added thereto under ice-cooling, and the mixture was stirred at room temperature for 4 hr. The reaction solution was concentrated under reduced pressure, the obtained residue was suspended in ethyl acetate, and the suspension was filtered through silica gel and Celite. The obtained filtrate was concentrated under reduced pressure to give (Z)-1-{5-[N'-(benzyloxy)carbamimidoyl]-3-chloropyridin-2-yl}ethane-1,2-diyl dimethanesulfonate as a crude product. Potassium hydroxide (powder, 0.11 g, 1.9 mmol) was suspended in dimethyl sulfoxide (2 mL), and then 2-(1H-imidazol-1-yl)acetonitrile (0.10 g, 0.94 mmol) and a dimethyl sulfoxide solution (2 mL) of carbon disulfide (72 mg, 0.94 mmol) were added thereto, and the mixture was stirred at room temperature for 2 hr. The obtained reaction solution was added to a dimethyl sulfoxide solution (4 mL) of (Z)-1-{5-[N'-(benzyloxy)carbamimidoyl]-3-chloropyridin-2-yl}ethane-1,2-diyl dimethanesulfonate, and the mixture was stirred at room temperature for 18 hr. Water was added thereto, and the mixture was extracted threetimes with ethyl acetate. The organic layer was dried over anhydrous sodium sulfate, and concentrated under reduced pressure. The obtained residue was purified by silica gel column chromatography to give (Z)-N'-(benzyloxy)-5-chloro-6-{(E)-2-[cyano(1H-imidazol-1-yl)methylene]-1,3-dithiolan-4-yl}nicotinimidamide (0.16 g).
yield: 55%
¹H-NMR(CDCl₃) δ value (ppm) :
   8.74(d,1H),8.03(d,1H),7.61(s,1H),7.44-7.30(m,H),7.17-7.15(m,1H),7.04-7.02(m,1H),5.68(dd,1H),5.15(s,2H),4.84(bs,2H),4.19(dd,1H),3.82(dd, 1H)

### Production Example 5 Production of ethyl (E)-(6-{2-[cyano(1H-1,2,4-triazol-1-yl)methylene]-1,3-dithiolan-4-yl}-5-fluoropyridin-3-yl)carbamate (Compound No.10)

### 5-1) Production of tertiary-butyl (5-fluoro-6-vinylpyridin-3-yl)carbamate

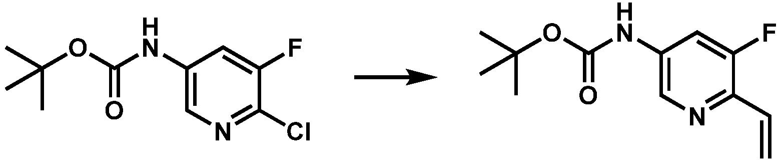

Tertiary-butyl (6-chloro-5-fluoropyridin-3-yl)carbamate (11 g, 46 mmol) was dissolved in 1,2-dimethoxyethane (150 mL) and water (100 mL), and then sodium carbonate (58 g,0.55 mol), [1,1'-bis(diphenylphosphino)ferrocene]palladium(II) dichloride dichloromethane adduct (5.6 g, 6.9 mmol) and potassium vinyltrifluoroborate (37 g,0.27mol) were added thereto, and the mixture was stirred with heating under reflux for 4.5 hr. Saturated aqueous sodium chloride solution and ethyl acetate were added thereto, and then the mixture was filtered, and the filtrate was extracted threetimes with ethyl acetate. The organic layer was dried over anhydrous sodium sulfate, and concentrated under reduced pressure. The obtained residue was purified by silica gel column chromatography to give tertiary-butyl (5-fluoro-6-vinylpyridin-3-yl)carbamate (9.9 g).
yield: 91%
¹H-NMR(CDCl₃) δ value (ppm) :
   8.09(d,1H),7.94(bd,1H),6.93(ddd,1H),6.62(bs,1H),6.26(dd,1H),5.48(d d,1H),1.53(s,9H)

### 5-2) Production of tertiary-butyl [6-(1,2-dihydroxyethyl)-5-fluoropyridin-3-yl]carbamate

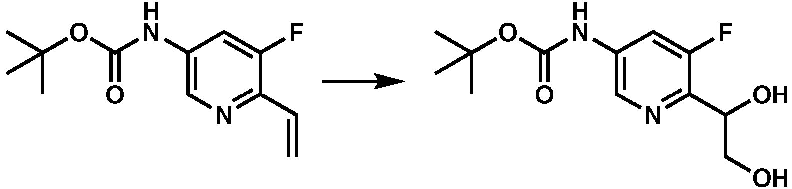

Tertiary-butyl (5-fluoro-6-vinylpyridin-3-yl)carbamate (9.9 g, 41 mmol) was dissolved in acetone (85 mL) and water (8.5 mL), and then 4-methylmorpholine N-oxide (50% aqueous solution, 19 g, 83 mmol) and osmium tetroxide (0.1M aqueous solution, 12 mL, 1.2 mmol) were added thereto, and the mixture was stirred at room temperature for 46 hr. Sodium dithionite (11 g, 62 mmol) and ethyl acetate were added thereto, and then the mixture was filtered through anhydrous sodium sulfate, silica gel and Celite, and the filtrate was concentrated under reduced pressure. The obtained residue was purified by silica gel column chromatography to give tertiary-butyl [6-(1,2-dihydroxyethyl)-5-fluoropyridin-3-yl]carbamate (7.3 g).
yield: 64%
¹H-NMR(CDCl₃) δ value (ppm) :
   8.14(d,1H),7.94(bd,1H),6.61(bs,1H),5.04-4.98(m,1H),4.20(d,1H),3.94(ddd,1H),3.74(ddd,1H),2.42(dd,1H),1.53(s , 9H)

### 5-3) Production of tertiary-butyl (E)-(6-{2-[cyano(1H-1,2,4-triazol-1-yl)methylene]-1,3-dithiolan-4-yl}-5-fluoropyridin-3-yl)carbamate

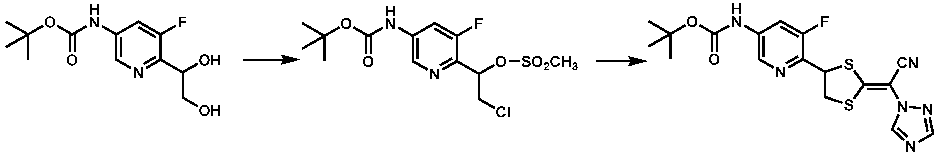

Tertiary-butyl [6-(1,2-dihydroxyethyl)-5-fluoropyridin-3-yl]carbamate (2.0 g, 7.4 mmol) and triethylamine (2.4 g, 24 mmol) were dissolved in chloroform (15 mL), and then methanesulfonyl chloride (2.2 g, 19 mmol) was added thereto under ice-cooling, and the mixture was stirred at room temperature for 4.5 hr. The reaction solution was concentrated under reduced pressure, the obtained residue was suspended in ethyl acetate, and the suspension was filtered through silica gel and Celite. The obtained filtrate was concentrated under reduced pressure to give 1-{5-[(tertiary-butoxycarbonyl)amino]-3-fluoropyridin-2-yl}-2-chloroethyl methanesulfonate as a crude product. Potassium hydroxide (powder, 1.2 g, 22 mmol) was suspended in dimethyl sulfoxide (8 mL), and then 2-(1H-1,2,4-triazol-1-yl)acetonitrile (1.2 g, 11 mmol) and a dimethyl sulfoxide solution (8 mL) of carbon disulfide (0.84 g, 11 mmol) were added thereto, and the mixture was stirred at room temperature for 2 hr. The obtained reaction solution was added to a dimethyl sulfoxide solution (16 mL) of 1-{5-[(tertiary-butoxycarbonyl)amino]-3-fluoropyridin-2-yl}-2-chloroethyl methanesulfonate, and the mixture was stirred at room temperature for 20 hr. Water was added thereto, and the mixture was extracted threetimes with ethyl acetate. The organic layer was dried over anhydrous sodium sulfate, and concentrated under reduced pressure. The obtained residue was purified by silica gel column chromatography to give tertiary-butyl (E)-(6-{2-[cyano(1H-1,2,4-triazol-1-yl)methylene]-1,3-dithiolan-4-yl}-5-fluoropyridin-3-yl)carbamate (0.84 g).
yield: 27%
melting point: 149-150°C

### 5-4) Production of (E)-2-[4-(5-amino-3-fluoropyridin-2-yl)-1,3-dithiolan-2-ylidene]-2-(1H-1,2,4-triazol-1-yl)acetonitrile

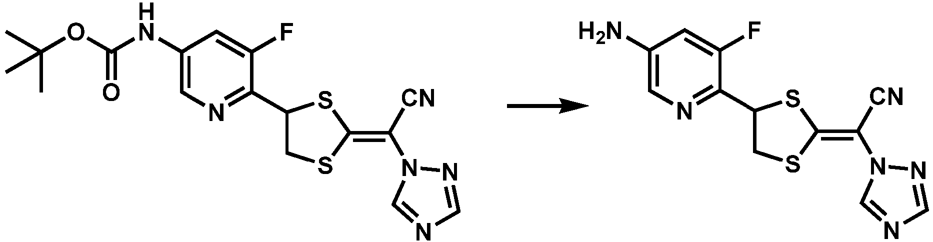

Tertiary-butyl (E)-(6-{2-[cyano(1H-1,2,4-triazol-1-yl)methylene]-1,3-dithiolan-4-yl}-5-fluoropyridin-3-yl)carbamate (0.50 g, 1.2 mmol) was dissolved in methanol (10 mL), and then the solution was added to 4M hydrogen chloride ethyl acetate solution (20 mL), and the mixture was stirred at room temperature for 52 hr. Saturated aqueous sodium hydrogencarbonate solution was added thereto, and the mixture was extracted threetimes with ethyl acetate. The organic layer was dried over anhydrous sodium sulfate, and concentrated under reduced pressure to give (E)-2-[4-(5-amino-3-fluoropyridin-2-yl)-1,3-dithiolan-2-ylidene]-2-(1H-1,2,4-triazol-1-yl)acetonitrile quantitatively.
melting point: 188-189°C

### 5-5) Production of ethyl (E)-(6-{2-[cyano(1H-1,2,4-triazol-1-yl)methylene]-1,3-dithiolan-4-yl}-5-fluoropyridin-3-yl)carbamate

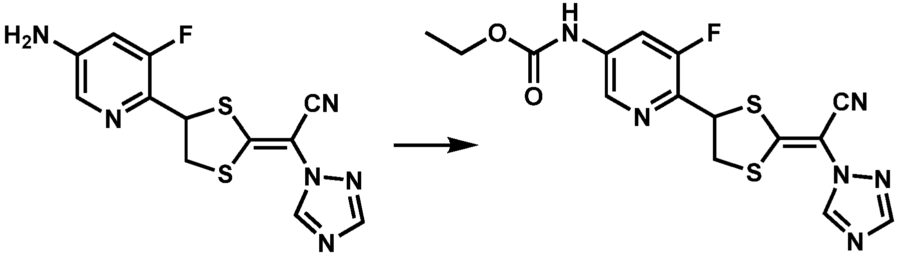

(E)-2-[4-(5-Amino-3-fluoropyridin-2-yl)-1,3-dithiolan-2-ylidene]-2-(1H-1,2,4-triazol-1-yl)acetonitrile (0.15 g, 0.47 mmol) was dissolved in pyridine (3 mL), and then ethyl chloroformate (61 mg, 0.56 mmol) was added thereto under ice-cooling, and the mixture was stirred at room temperature for 2.5 hr. Saturated aqueous sodium hydrogencarbonate solution was added thereto, and the mixture was extracted threetimes with ethyl acetate. The organic layer was dried over anhydrous sodium sulfate, and concentrated under reduced pressure. The obtained residue was suspended in methyl tertiary-butyl ether, and the solid was filtered. The filtered solid was air-dried to give ethyl (E)-(6-{2-[cyano(1H-1,2,4-triazol-1-yl)methylene]-1,3-dithiolan-4-yl}-5-fluoropyridin-3-yl)carbamate (0.15 g).
yield: 81%
melting point: 160-161°C

### Production Example 6 Production of ethyl (R,E)-(6-{2-[cyano(1H-1,2,4-triazol-1-yl)methylene]-1,3-dithiolan-4-yl}-5-fluoropyridin-3-yl)carbamate (Compound No.8)

### 6-1) Production of (R,E)-2-[4-(5-amino-3-fluoropyridin-2-yl)-1,3-dithiolan-2-ylidene]-2-(1H-1,2,4-triazol-1-yl)acetonitrile

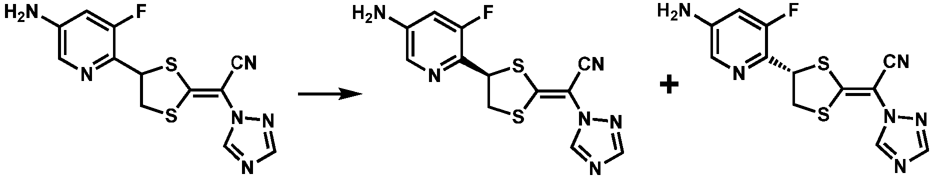

(E)-2-[4-(5-Amino-3-fluoropyridin-2-yl)-1,3-dithiolan-2-ylidene]-2-(1H-1,2,4-triazol-1-yl)acetonitrile (15 g, 47 mmol) obtained in 5-4) above was purified by supercritical fluid chromatography (column: CHIRALPAK AD manufactured by Daicel (250 mm × 50 mm, 10 µm), mobile phase: 0.1% ammonia methanol solution) to give (R,E)-2-[4-(5-amino-3-fluoropyridin-2-yl)-1,3-dithiolan-2-ylidene]-2-(1H-1,2,4-triazol-1-yl)acetonitrile (5.0 g, yield: 67%, melting point: 215-216°C, [α]D25:+79° (1.1 g/100 mL, methanol)) and (S,E)-2-[4-(5-amino-3-fluoropyridin-2-yl)-1,3-dithiolan-2-ylidene]-2-(1H-1,2,4-triazol-1-yl)acetonitrile (5.0 g, yield: 67%, melting point: 215-216°C, [α]D25:-60° (1.2 g/100 mL, methanol)).

### 6-2) Production of ethyl (R,E)-(6-{2-[cyano(1H-1,2,4-triazol-1-yl)methylene]-1,3-dithiolan-4-yl}-5-fluoropyridin-3-yl)carbamate

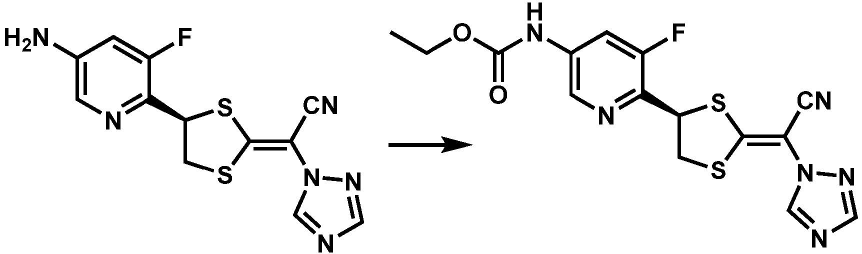

(R,E)-2-[4-(5-Amino-3-fluoropyridin-2-yl)-1,3-dithiolan-2-ylidene]-2-(1H-1,2,4-triazol-1-yl)acetonitrile (0.10 g, 0.31 mmol) was dissolved in pyridine (2 mL), and then ethyl chloroformate (41 mg, 0.38 mmol) was added thereto under ice-cooling, and the mixture was stirred at room temperature for 4 hr. The reaction solution was concentrated under reduced pressure, and the obtained residue was purified by silica gel column chromatography to give ethyl (R,E)-(6-{2-[cyano(1H-1,2,4-triazol-1-yl)methylene]-1,3-dithiolan-4-yl}-5-fluoropyridin-3-yl)carbamate (0.12 g).
yield: 97%
melting point: 177-178°C

Formulation Examples are shown below.

| Formulation Example 1. | |
|---|---|
| compound of the present invention | 10 parts |
| magnesium stearate | 10 parts |
| lactose | 80 parts |

The above components were mixed uniformly to give a powder or fine granules.

### Formulation Example 2.

| | |
|---|---|
| compound of the present invention | 20 parts |
| starch | 10 parts |
| lactose | 15 parts |
| ethyl cellulose | 20 parts |
| polyvinyl alcohol | 5 parts |
| water | 30 parts |

The above components were mixed uniformly, crushed, granulated and sieved to give granules.

### Formulation Example 3.

| | |
|---|---|
| compound of the present invention | 0.5 parts |
| nonionic surfactant | 2.5 parts |
| saline | 97 parts |

The above components were mixed under warming and sterilized to give an injection.

### Formulation Example 4.

| | |
|---|---|
| compound of the present invention | 0.01 parts |
| 0.5% carboxymethyl cellulose | 99.99 parts |

The above components were suspended to give a suspension.

### Formulation Example 5.

| | |
|---|---|
| compound of the present invention | 1 parts |
| polyethylene glycol 400 | 99 parts |

The above components were mixed and dissolved to give a liquid for application.

### Formulation Example 6.

| | |
|---|---|
| compound of the present invention | 2 parts |
| polyethylene glycol 400 | 49 parts |
| polyethylene glycol 4000 | 49 parts |

The above components were mixed and dissolved under warming, and cooled to give an ointment.

### Formulation Example 7.

| | |
|---|---|
| compound of the present invention | 3 parts |
| 1,2-propanediol | 5 parts |
| glycerol stearate | 5 parts |
| whale wax | 5 parts |
| isopropyl myristate | 10 parts |
| polysorbate | 4 parts |

The above components were mixed under warming, cooled, and 68 parts of water was added thereto with stirring to give a cream.

### Formulation Example 8.

One part of the compound of the present invention, 5 parts of benzyl alcohol, 30 parts of ethanol, and 47 parts of propylene glycol were mixed and dissolved, and then an aqueous solution consisting of 1 part of Hiviskowa 104 and 15 parts of purified water was added to this solution to give a uniform solution. Then, diisopropanolamine was added thereto under stirring to give a gel.

### Formulation Example 9.

One part of the compound of the present invention was dissolved in 5 parts of benzyl alcohol and 5 parts of diethyl sebacate, and 5 parts of ethyl alcohol, 6 parts of stearyl alcohol, 1 part of sorbitan monostearate and 8 parts of polyoxyethylene monostearate were added thereto, and dissolved with warming to 70°C. While maintaining the obtained uniform solution at 70°C, 69 parts of purified water warmed to 70°C was added thereto, and then the mixture was cooled with continued stirring to give a cream.

Next, Experimental Examples are shown below.

### Experimental Example 1. in vitro anti-Aspergillus activity test

Aspergillus fumigatus TIMM0063 was used as the strain. The test fungi were cultured on a Potato dextrose agar slant medium at 35°C for 4 days, and then sterilized saline containing 0.1% (v/v) Tween 80 was added to release the conidia. This conidial suspension was filtered through sterile gauze, and the number of conidia was counted using a hemocytometer. The same solution was adjusted to a conidial concentration of 1×10⁶ conidia/mL, and this was used as an inoculum. Each compound was dissolved in dimethyl sulfoxide to a predetermined concentration, and diluted 50-fold with 0.165 M MOPS buffered RPMI 1640 liquid medium, and each diluted solution was dispensed into a 96-well microplate by each 100 µL. The inoculum was diluted 50-fold with 0.165 M MOPS buffered RPMI 1640 liquid medium containing 20% alamar Blue^{™}, and the diluted solution was inoculated into the 96-well microplate containing the compound by each 100 µL (final inoculum amount 1.0×10⁴ conidia/mL). After static culture at 35°C for 48 hr, the degree of color development of the alamar Blue^{™} was measured using a microplate reader (O.D. 570-595 nm), and the minimum concentration showing 80% or more inhibition was defined as the minimum inhibitory concentration (MIC). The results are shown in Tables 22 to 25.

### Experimental Example 2. in vitro anti-Candida activity test

Candida albicans NBRC1270 was used as the strain. The test fungi were cultured on a Sabouraud dextrose agar slant medium at 35°C for 24 hr, and then suspended in sterilized saline. The number of cells in this suspension was counted using a hemocytometer, and the same suspension was adjusted to a cell concentration of 1.5×10⁵ cells/mL, and this was used as an inoculum. Each compound was dissolved in dimethyl sulfoxide to a predetermined concentration, and diluted 50-fold with 0.165 M MOPS buffered RPMI 1640 liquid medium, and each diluted solution was dispensed into a 96-well microplate by each 100 µL. The inoculum was diluted 50-fold with 0.165 M MOPS buffered RPMI 1640 liquid medium containing 20% alamar Blue^{™}, and the diluted solution was inoculated into the 96-well microplate containing the compound by each 100 µL (final inoculum amount 1.5×10³ cells/mL). After static culture at 35°C for 24 hr, the degree of color development of the alamar Blue^{™} was measured using a microplate reader (O.D. 570-595 nm), and the minimum concentration showing 80% or more inhibition was defined as the minimum inhibitory concentration (MIC). The results are shown in Tables 22 to 25.

### Experimental Example 3. in vitro anti-tinea fungus activity test

Trichophyton rubrum NBRC6203 and Trichophyton mentagrophytes TIMM2789 were used as the strains. The test fungi were cultured on a Sabouraud dextrose agar slant medium at 27°C for 10-13 days, and then suspended in sterilized saline. The number of cells in this suspension was counted using a hemocytometer, and the same suspension was adjusted to a cell concentration of 2×10⁶ conidia/mL, and this was used as an inoculum. Each compound was dissolved in dimethyl sulfoxide to a predetermined concentration, and diluted 50-fold with 0.165 M MOPS buffered RPMI 1640 liquid medium, and each diluted solution was dispensed into a 96-well microplate by each 100 µL. The inoculum was diluted 50-fold with 0.165 M MOPS buffered RPMI 1640 liquid medium containing 20% alamar Blue^{™}, and the diluted solution was inoculated into the 96-well microplate containing the compound by each 100 µL (final inoculum amount 2×10⁴ conidia/mL). After static culture at 27°C for 3 days (Trichophyton mentagrophytes) or for 5 days (Trichophyton rubrum), the degree of color development of the alamar Blue^{™} was measured using a microplate reader (O.D. 570-595 nm), and the minimum concentration showing 80% or more inhibition was defined as the minimum inhibitory concentration (MIC). The results are shown in Table 22.

**[Table 22]**

| minimum inhibitory concentration against various fungi (MIC, µg/ml) | | | | |
|---|---|---|---|---|
| Compound No. | A. fumigatus | C. albicans | T. rubrum | T. mentagrophytes |
| 1 | 0.0013 | 0.1 | ≦0.001 | ≦0.001 |
| 2 | 0.01 | 1 | 0.01 | 0.01 |
| 3 | 0.0025 | 0.1 | ≦0.001 | ≦0.001 |
| 4 | 0.005 | 1 | 0.01 | 0.01 |
| 5 | 0.01 | 0.005 | - | - |
| 6 | 0.005 | 0.01 | 0.01 | 0.01 |
| 7 | 0.005 | 0.01 | ≦0.001 | 0.01 |
| 8 | 0.005 | 0.025 | - | - |
| 9 | 0.005 | 0.013 | - | - |
| 10 | 0.025 | 0.05 | 0.01 | 0.01 |
| 11 | 0.005 | 0.01 | ≦0.001 | 0.01 |
| 12 | 0.1 | 0.01 | 0.01 | 0.1 |
| 13 | 0.005 | 0.0025 | - | - |
| 14 | 0.005 | 0.0013 | - | - |
| 15 | 0.005 | 0.0025 | 0.01 | 0.01 |
| 16 | 0.005 | 0.0025 | ≦0.001 | 0.01 |
| 17 | 0.005 | 0.0013 | - | - |
| 18 | 0.01 | 0.05 | - | - |
| 19 | 0.005 | 0.01 | ≦0.001 | 0.01 |
| 20 | 0.01 | 0.013 | - | - |
| 21 | 0.005 | 0.005 | ≦0.001 | 0.01 |
| 22 | 0.005 | 0.0006 | - | - |
| 23 | 0.005 | 0.0006 | ≦0.001 | 0.01 |
| 24 | 0.0025 | 0.0025 | ≦0.001 | ≦0.001 |
| 25 | 0.0025 | 0.0006 | ≦0.001 | ≦0.001 |
| 26 | 0.005 | 0.0013 | - | - |
| 27 | 0.005 | 0.0013 | ≦0.001 | ≦0.001 |

In the table, "-" indicates that the test was not performed.

**[Table 23]**

| Compound No. | A. fumigatus | C. albicans |
|---|---|---|
| 28 | 0.01 | 0.01 |
| 66 | 0.01 | 0.005 |
| 67 | 0.01 | 0.0025 |
| 73 | 0.01 | 0.01 |
| 74 | 0.01 | 0.01 |
| 75 | 0.01 | 0.01 |
| 79 | 0.005 | 0.005 |
| 83 | 0.01 | 0.005 |
| 84 | 0.005 | 0.005 |
| 87 | 0.005 | 0.00125 |
| 89 | 0.01 | 0.0025 |
| 92 | 0.0025 | 0.00063 |
| 94 | 0.0025 | 0.0025 |
| 96 | 0.01 | 0.005 |
| 97 | 0.01 | 0.0025 |
| 98 | 0.005 | 0.0025 |
| 99 | 0.005 | 0.0025 |
| 100 | 0.0025 | 0.00063 |
| 103 | 0.01 | 0.005 |
| 106 | 0.005 | 0.00125 |
| 107 | 0.01 | 0.005 |
| 110 | 0.005 | 0.01 |
| 112 | 0.005 | 0.0025 |

**[Table 24]**

| Compound No. | A. fumigatus | C. albicans |
|---|---|---|
| 114 | 0.01 | 0.01 |
| 115 | 0.01 | 0.005 |
| 118 | 0.005 | 0.00125 |
| 128 | 0.01 | 0.01 |
| 130 | 0.005 | 0.0025 |
| 131 | 0.01 | 0.01 |
| 139 | 0.0025 | 0.00125 |
| 140 | 0.0025 | 0.0025 |
| 141 | 0.0025 | 0.0025 |
| 142 | 0.01 | 0.0025 |
| 143 | 0.005 | 0.0025 |
| 144 | 0.005 | 0.01 |

**[Table 25]**

| Compound No. | A. fumigatus | C. albicans |
|---|---|---|
| 147 | 0.005 | 0.0025 |
| 149 | 0.01 | 0.005 |
| 150 | 0.01 | 0.005 |
| 151 | 0.01 | 0.0025 |
| 152 | 0.005 | 0.00125 |
| 154 | 0.01 | 0.0025 |
| 155 | 0.01 | 0.0025 |
| 156 | 0.005 | 0.0025 |
| 157 | 0.005 | 0.005 |
| 158 | 0.005 | 0.00125 |
| 159 | 0.005 | 0.005 |
| 162 | 0.005 | 0.0025 |
| 163 | 0.0025 | 0.0025 |

From the results in Tables 22 to 25, it is seen that the compound represented by the general formula (I) of the present invention has superior antifungal activity, and is useful as a therapeutic agent for fungal infections such as localized fungal infections, systemic fungal infections and the like, caused by Trichophyton, Candida, Aspergillus and the like.

### [Industrial Applicability]

According to the present invention, the azole compound represented by the general formula (I) or a salt thereof has superior antifungal activity against fungi such as Trichophyton, Candida, Aspergillus and the like, and therefore can be used as a therapeutic agent for fungal infections such as localized fungal infections, systemic fungal infections and the like, caused by the fungi.

This application is based on patent applications No. 2022-043359 filed on March 18, 2022 and No. 2022-190422 filed on November 29, 2022 in Japan, the contents of which are encompassed in full herein.

## Claims

1. A compound represented by the general formula (I) 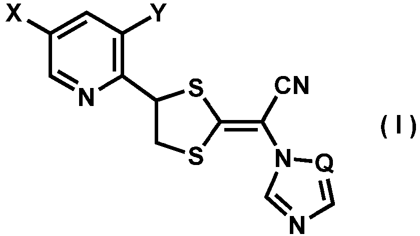 wherein
X is a halogen atom, a cyano group, an amino group, a hydroxy group, a formyl group, a cyano C₁₋₆ alkyl group, a hydroxy C₁₋₆ alkyl group, a halo C₁₋₆ alkyl group, a C₂₋₆ alkenyl group, a C₂₋₆ alkynyl group, a C₁₋₆ alkoxy group, a halo C₁₋₆ alkoxy group, a C₂₋₆ alkenyloxy group, a C₂₋₆ alkynyloxy group, a C₁₋₆ alkoxy C₁₋₆ alkoxy group, a cyano C₁₋₆ alkoxy group, a C₁₋₆ alkyl-carbonyl group, a mono (C₁₋₆ alkyl-carbonyl) amino group, a mono (halo C₁₋₆ alkyl-carbonyl) amino group, a mono (phenyl C₁₋₆ alkyl-carbonyl) amino group, a tetrahydro-2H-pyran-2-yloxy group, a tetrahydro-2H-pyran-2-yloxymethyl group, a dioxolanyl group, a substituted dioxolanyl group having 1 to 5 substituents each independently selected from substituent group Z, a 2-oxo-1,3-dioxolan-4-yl group, an isoxazolinyl group, a substituted isoxazolinyl group having 1 to 4 substituents each independently selected from substituent group Z, a -N(R¹)CO₂R² group wherein R¹ is a hydrogen atom or a C₁₋₆ alkyl group, and R² is a C₁₋₆ alkyl group, a cyano C₁₋₆ alkyl group, a C₃₋₆ cycloalkyl group, a C₁₋₆ haloalkyl group, a C₁₋₆ alkoxy C₁₋₆ alkyl group, a C₃₋₆ cycloalkyl C₁₋₆ alkyl group, a C₁₋₆ alkoxycarbonylamino C₃₋₆ cycloalkyl C₁₋₆ alkyl group, a C₁₋₆ alkyl C₃₋₆ cycloalkyl C₁₋₆ alkyl group, a C₂₋₆ alkynyl C₃₋₆ cycloalkyl C₁₋₆ alkyl group, a cyano C₃₋₆ cycloalkyl C₁₋₆ alkyl group, an oxo C₃₋₆ cycloalkyl C₁₋₆ alkyl group, an oxotetrahydrofuranyl C₁₋₆ alkyl group, a halo C₃₋₆ cycloalkyl C₁₋₆ alkyl group, an oxetanyl C₁₋₆ alkyl group, a (3-oxabicyclo[3.1.0]hexan-6-yl)methyl group, a pyridyl C₁₋₆ alkyl group, a substituted pyridyl C₁₋₆ alkyl group having, on a ring, 1 to 4 substituents each independently selected from substituent group Z, a pyrazolyl C₁₋₆ alkyl group, a substituted pyrazolyl C₁₋₆ alkyl group having, on a ring, 1 to 3 substituents each independently selected from substituent group Z, a phenyl C₃₋₆ cycloalkyl C₁₋₆ alkyl group, a phenyl C₁₋₆ alkyl group, or a substituted phenyl C₁₋₆ alkyl group having, on a ring, 1 to 5 substituents each independently selected from substituent group Z, or a -C(R³)=NOR⁴ group wherein R³ is a hydrogen atom, an amino group or a C₁₋₆ alkyl group, and R⁴ is a hydrogen atom, a C₁₋₆ alkyl group, a C₃₋₆ cycloalkyl C₁₋₆ alkyl group, a halo C₃₋₆ cycloalkyl C₁₋₆ alkyl group, a phenyl C₁₋₆ alkyl group, or a substituted phenyl C₁₋₆ alkyl group having, on a ring, 1 to 5 substituents each independently selected from substituent group Z,
Y is a hydrogen atom, a halogen atom, a cyano group, or a C₁₋₆ alkyl group,
Q is CH or a nitrogen atom, and
substituent group Z comprises a halogen atom, a nitro group, a cyano group, a C₁₋₆ alkyl group, a halo C₁₋₆ alkyl group, a C₁₋₆ alkoxy group, a halo C₁₋₆ alkoxy group, a C₁₋₆ alkylsulfanyl group, a C₁₋₆ alkylsulfinyl group, a C₁₋₆ alkylsulfonyl group, a mono (C₁₋₆ alkyl-carbonyl) amino group, a C₁₋₆ alkoxy-carbonyl group, a C₁₋₆ alkyl-carbonyl group, and a mono(C₁₋₆ alkyl)aminocarbonyl group, or a salt thereof.

2. The compound or salt thereof according to claim 1, wherein X is a -N(R¹)CO₂R² group wherein R¹ is a hydrogen atom or a C₁₋₆ alkyl group, and R² is a C₁₋₆ alkyl group, a C₃₋₆ cycloalkyl group, a C₃₋₆ cycloalkyl C₁₋₆ alkyl group, a halo C₃₋₆ cycloalkyl C₁₋₆ alkyl group, a phenyl C₁₋₆ alkyl group, or a substituted phenyl C₁₋₆ alkyl group having, on a ring, 1 to 5 substituents each independently selected from substituent group Z (substituent group Z is as defined in claim 1), or a -C(R³)=NOR⁴ group wherein R³ is a hydrogen atom, an amino group or a C₁₋₆ alkyl group, and R⁴ is a C₁₋₆ alkyl group, a C₃₋₆ cycloalkyl C₁₋₆ alkyl group, a halo C₃₋₆ cycloalkyl C₁₋₆ alkyl group, a phenyl C₁₋₆ alkyl group, or a substituted phenyl C₁₋₆ alkyl group having, on a ring, 1 to 5 substituents each independently selected from substituent group Z (substituent group Z is as defined in claim 1), and
Y is a halogen atom.

3. The compound or salt thereof according to claim 1, wherein X is a -N(R¹)CO₂R² group wherein R¹ is a hydrogen atom, and R² is a C₁₋₆ alkyl group, a C₃₋₆ cycloalkyl C₁₋₆ alkyl group, a halo C₃₋₆ cycloalkyl C₁₋₆ alkyl group, or a phenyl C₁₋₆ alkyl group, or a - C(R³)=NOR⁴ group wherein R³ is a hydrogen atom, and R⁴ is a C₁₋₆ alkyl group, a C₃₋₆ cycloalkyl C₁₋₆ alkyl group, a halo C₃₋₆ cycloalkyl C₁₋₆ alkyl group, or a phenyl C₁₋₆ alkyl group,
Y is a halogen atom, and
Q is a nitrogen atom.

4. A pharmaceutical composition comprising the compound or salt thereof according to claim 1, and a pharmaceutically acceptable inert carrier or diluent.

5. An antifungal agent comprising the compound or salt thereof according to claim 1 as an active ingredient.

6. A therapeutic agent for a fungal infection, comprising the compound or salt thereof according to claim 1 as an active ingredient.

7. A method for inhibiting fungal growth in a mammal, comprising administering to the mammal a pharmacologically effective amount of the compound or salt thereof according to claim 1.

8. A method for treating a fungal infection in a mammal, comprising administering to the mammal a pharmacologically effective amount of the compound or salt thereof according to claim 1.

9. The compound or salt thereof according to claim 1, for use in inhibiting fungal growth.

10. The compound or salt thereof according to claim 1, for use in the treatment of a fungal infection.

11. Use of the compound or salt thereof according to claim 1, for the manufacture of an antifungal agent.

12. Use of the compound or salt thereof according to claim 1, for the manufacture of a therapeutic agent for a fungal infection.
